# EUROPEAN PATENT APPLICATION

(11) **EP 1 245 671 A1**
(43) Date of publication of application: **02.10.2002**
(21) Application number: 01302821.2
(22) Date of filing: 27.03.2001
(51) Int. Cl.: C12N 9/12

(54) **NUCLEIC ACIDS ENCODING HUMAN TELOMERASE SUBUNITS DISPLAYING HOMOLOGY TO YEAST EST1**

(71) Applicant: BTG INTERNATIONAL LIMITED, London EC4M 7SB (GB)
(72) Inventor: The designation of the inventor has not yet been filed
(74) Representative: Dolan, Anthony Patrick

(57) **Abstract**

Isolated, enriched, purified or recombinant nucleic acids are provided which encode for a protein having activity as a human telomerase activity modulator characterised in that they comprise a nucleotide sequence encoding for an amino acid sequence selected from those of SEQ ID No 2, SEQ ID No 4, SEQ ID No 5 and SEQ ID No 6 and sequences having at least 70% amino acid homology thereto and an active peptide fragment of any of these sequences.

The encoded proteins act as human EST-1 proteins and have use in modulating human telomerase and in screening for modulators of human telomerase.

SEQUENCE LISTING

<110> Swiss Institute for Experimental Cancer Research

<120> TELOMERE FORMATION MODULATING PROTEIN

<130> TELOMERE FORMATION MODULATING PROTEIN

<140>
<141>

<160> 8

<170> PatentIn Ver. 2.1

<210> 1
<211> 4260
<212> DNA
<213> Homo sapiens

<220>
<221> CDS
<222> (1)..(4260)

<400> 1
atg gcg gaa ggg ctg gag cgt gtg cgg atc tcc gcg tcg gag ctg cgc    48
Met Ala Glu Gly Leu Glu Arg Val Arg Ile Ser Ala Ser Glu Leu Arg
  1               5                  10                  15

ggg atc ctg gct act ctg gcc ccg cag gcc ggg agc aga gaa aac atg    96
Gly Ile Leu Ala Thr Leu Ala Pro Gln Ala Gly Ser Arg Glu Asn Met
             20                  25                  30

aag gaa tta aag gag gcc agg ccg cgc aaa gat aac agg cgt cct gat    144
Lys Glu Leu Lys Glu Ala Arg Pro Arg Lys Asp Asn Arg Arg Pro Asp
         35                  40                  45

ctg gaa atc tat aag cct ggc ctt tct cgg cta agg aac aag ccc aaa    192
Leu Glu Ile Tyr Lys Pro Gly Leu Ser Arg Leu Arg Asn Lys Pro Lys
     50                  55                  60

atc aag gaa ccc cct ggg agt gag gag ttc aaa gat gaa att gtt aat    240
Ile Lys Glu Pro Pro Gly Ser Glu Glu Phe Lys Asp Glu Ile Val Asn
 65                  70                  75                  80

gac cga gat tgc tct gct gtt gaa aat ggt aca cag ccc gtt aaa gat    288
Asp Arg Asp Cys Ser Ala Val Glu Asn Gly Thr Gln Pro Val Lys Asp
                 85                  90                  95

gtc tgc aag gaa ctg aac aac caa gag cag aat ggt cct ata gac ccg    336
Val Cys Lys Glu Leu Asn Asn Gln Glu Gln Asn Gly Pro Ile Asp Pro
            100                 105                 110

gaa aat aat cgg gga caa gaa tcc ttt cct agg act gct gga caa gag    384
Glu Asn Asn Arg Gly Gln Glu Ser Phe Pro Arg Thr Ala Gly Gln Glu
        115                 120                 125

gat cgt agt cta aaa att atc aaa aga aca aag aaa ccc gac ctg cag    432
Asp Arg Ser Leu Lys Ile Ile Lys Arg Thr Lys Lys Pro Asp Leu Gln
    130                 135                 140

atc tat cag cct gga cga cgt ttg cag act gtt agc aaa gaa tcc gcc     480
Ile Tyr Gln Pro Gly Arg Arg Leu Gln Thr Val Ser Lys Glu Ser Ala
145                 150                 155                 160

agt cgg gtg gag gag gaa gaa gtc ctc aac cag gta gaa caa ctg aga     528
Ser Arg Val Glu Glu Glu Glu Val Leu Asn Gln Val Glu Gln Leu Arg
                165                 170                 175

gta gag gaa gat gag tgt agg gga aat gtt gcg aag gag gaa gtt gcg     576
Val Glu Glu Asp Glu Cys Arg Gly Asn Val Ala Lys Glu Glu Val Ala
            180                 185                 190

aat aaa cca gac agg gcc gag ata gaa aag agc cca ggt ggt ggg aga     624
Asn Lys Pro Asp Arg Ala Glu Ile Glu Lys Ser Pro Gly Gly Gly Arg
        195                 200                 205

gta ggg gct gca aaa gga gaa aaa gga aag agg atg gga aaa ggg gag     672
Val Gly Ala Ala Lys Gly Glu Lys Gly Lys Arg Met Gly Lys Gly Glu
    210                 215                 220

ggg gtg agg gaa acc cac gac gac ccg gcc cgc ggg agg ccg ggc tcc     720
Gly Val Arg Glu Thr His Asp Asp Pro Ala Arg Gly Arg Pro Gly Ser
225                 230                 235                 240

gca aag cgc tac tcc cgc tca gac aaa cga agg aat cgc tac cgc acg     768
Ala Lys Arg Tyr Ser Arg Ser Asp Lys Arg Arg Asn Arg Tyr Arg Thr
                245                 250                 255

cgc agc acc agc tca gct ggc agc aac aac agc gct gag gga gct ggc     816
Arg Ser Thr Ser Ser Ala Gly Ser Asn Asn Ser Ala Glu Gly Ala Gly
            260                 265                 270

ctg acg gat aat gga tgt cgc cgc cgc cga cag gat agg acc aag gag     864
Leu Thr Asp Asn Gly Cys Arg Arg Arg Arg Gln Asp Arg Thr Lys Glu
        275                 280                 285

agg cca cga ctg aag cag caa gtg tct gtg tcc tca acc gat tcc tta     912
Arg Pro Arg Leu Lys Gln Gln Val Ser Val Ser Ser Thr Asp Ser Leu
    290                 295                 300

gac gag gac aga att gat gag cct gat gga tta gga ccc agg aga agt     960
Asp Glu Asp Arg Ile Asp Glu Pro Asp Gly Leu Gly Pro Arg Arg Ser
305                 310                 315                 320

tca gaa agg aag aga cat tta gaa aga aac tgg tct ggc cgt ggg gag     1008
Ser Glu Arg Lys Arg His Leu Glu Arg Asn Trp Ser Gly Arg Gly Glu
                325                 330                 335

ggt gag cag aaa acc agt gct aaa gaa tat cga ggc act ctt cgt gtc     1056
Gly Glu Gln Lys Thr Ser Ala Lys Glu Tyr Arg Gly Thr Leu Arg Val
            340                 345                 350

act ttc gat gca gaa gcc atg aac aaa gag tct ccc atg gtg agg tca     1104
Thr Phe Asp Ala Glu Ala Met Asn Lys Glu Ser Pro Met Val Arg Ser
        355                 360                 365

gcc agg gat gat atg gat aga gga aag cct gac aaa ggc ttg agc agt     1152
Ala Arg Asp Asp Met Asp Arg Gly Lys Pro Asp Lys Gly Leu Ser Ser
    370                 375                 380

ggg ggc aaa ggc tct gag aag cag gag tcc aaa aac ccg aaa caa gaa    1200
Gly Gly Lys Gly Ser Glu Lys Gln Glu Ser Lys Asn Pro Lys Gln Glu
385                 390                 395                 400

ctt cgg ggt cgt ggt cgt ggc att ctg att ttg cct gcc cat acc acc    1248
Leu Arg Gly Arg Gly Arg Gly Ile Leu Ile Leu Pro Ala His Thr Thr
                405                 410                 415

cta tct gtc aat tca gca ggt tct cca gag tcc gcg cct ttg gga cct    1296
Leu Ser Val Asn Ser Ala Gly Ser Pro Glu Ser Ala Pro Leu Gly Pro
            420                 425                 430

cgg ctt ttg ttt gga tct ggt agt aag ggg tct cgg agt tgg ggc cgt    1344
Arg Leu Leu Phe Gly Ser Gly Ser Lys Gly Ser Arg Ser Trp Gly Arg
        435                 440                 445

gga ggc acc aca cgc cga ttg tgg gac cca aac aat cct gat cag aaa    1392
Gly Gly Thr Thr Arg Arg Leu Trp Asp Pro Asn Asn Pro Asp Gln Lys
    450                 455                 460

cct gct cta aag act cat acg ccc cag cta cat ttc ttg gac act gat    1440
Pro Ala Leu Lys Thr His Thr Pro Gln Leu His Phe Leu Asp Thr Asp
465                 470                 475                 480

gat gaa gtc agc cct aca tct tgg ggt gac tca cgc cag gct cag gca    1488
Asp Glu Val Ser Pro Thr Ser Trp Gly Asp Ser Arg Gln Ala Gln Ala
                485                 490                 495

tct tac tat aag ttt caa aac tct gac aac ccc tat tat tac ccc cgg    1536
Ser Tyr Tyr Lys Phe Gln Asn Ser Asp Asn Pro Tyr Tyr Tyr Pro Arg
            500                 505                 510

aca cca ggc cct gcc tcc cag tat ccc tat acg ggc tat aac cct cta    1584
Thr Pro Gly Pro Ala Ser Gln Tyr Pro Tyr Thr Gly Tyr Asn Pro Leu
        515                 520                 525

cag tac cca gtg ggc cct acg aat ggt gtg tac cca ggg cct tac tac    1632
Gln Tyr Pro Val Gly Pro Thr Asn Gly Val Tyr Pro Gly Pro Tyr Tyr
    530                 535                 540

cca ggc tac ccg act ccg tca gga cag tat gtg tgt agc cct ctg cct    1680
Pro Gly Tyr Pro Thr Pro Ser Gly Gln Tyr Val Cys Ser Pro Leu Pro
545                 550                 555                 560

acc agc acc atg agt ccc gag gag gta gag cag cac atg agg aac ctg    1728
Thr Ser Thr Met Ser Pro Glu Glu Val Glu Gln His Met Arg Asn Leu
                565                 570                 575

cag caa cag gag ctg cac agg ctt ctc cgg gtg gct gac aac cag gaa    1776
Gln Gln Gln Glu Leu His Arg Leu Leu Arg Val Ala Asp Asn Gln Glu
            580                 585                 590

ctg cag ctc agc aac ctg ctc tcc agg gac cgc atc agt ccg gag ggc    1824
Leu Gln Leu Ser Asn Leu Leu Ser Arg Asp Arg Ile Ser Pro Glu Gly
        595                 600                 605

ctg gag aag atg gcg caa ctc aga gct gaa ctg ctg cag cta tat gag    1872
Leu Glu Lys Met Ala Gln Leu Arg Ala Glu Leu Leu Gln Leu Tyr Glu
    610                 615                 620

cgc tgt att cta tta gat att gag ttc tct gat aat cag aat gtg gat    1920

Arg Cys Ile Leu Leu Asp Ile Glu Phe Ser Asp Asn Gln Asn Val Asp
625                 630                 635                 640

cag atc ctg tgg aag aat gct ttc tat cag gtg att gag aag ttc agg     1968
Gln Ile Leu Trp Lys Asn Ala Phe Tyr Gln Val Ile Glu Lys Phe Arg
                645                 650                 655

caa ctt gtc aag gat ccg aat gtt gag aac cca gaa cag att cgg aac     2016
Gln Leu Val Lys Asp Pro Asn Val Glu Asn Pro Glu Gln Ile Arg Asn
            660                 665                 670

aga ctt ttg gag ctc ttg gat gag ggt agt gac ttc ttt gat agt ttg     2064
Arg Leu Leu Glu Leu Leu Asp Glu Gly Ser Asp Phe Phe Asp Ser Leu
        675                 680                 685

ctt cag aag ctg cag gtt act tac aag ttc aaa ctg gaa gac tac atg     2112
Leu Gln Lys Leu Gln Val Thr Tyr Lys Phe Lys Leu Glu Asp Tyr Met
    690                 695                 700

gat ggt ctt gcc att cgc agc aag cca tta cgc aag aca gta aaa tat     2160
Asp Gly Leu Ala Ile Arg Ser Lys Pro Leu Arg Lys Thr Val Lys Tyr
705                 710                 715                 720

gcc ttg atc agt gcc cag cga tgc atg ata tgc caa gga gat att gct     2208
Ala Leu Ile Ser Ala Gln Arg Cys Met Ile Cys Gln Gly Asp Ile Ala
                725                 730                 735

agg tac cgg gag caa gcc agt gat aca gcg aat tat ggg aaa gca cgc     2256
Arg Tyr Arg Glu Gln Ala Ser Asp Thr Ala Asn Tyr Gly Lys Ala Arg
            740                 745                 750

agt tgg tac ctg aag gcc cag cac att gct ccc aag aat ggg cgc ccc     2304
Ser Trp Tyr Leu Lys Ala Gln His Ile Ala Pro Lys Asn Gly Arg Pro
        755                 760                 765

tat aac cag ttg gct ttg ctg gca gtg tat acg agg agg aag ctt gac     2352
Tyr Asn Gln Leu Ala Leu Leu Ala Val Tyr Thr Arg Arg Lys Leu Asp
    770                 775                 780

gct gtc tat tac tat atg cgc agt tta gct gcc agc aac cct atc ctg     2400
Ala Val Tyr Tyr Tyr Met Arg Ser Leu Ala Ala Ser Asn Pro Ile Leu
785                 790                 795                 800

act gcc aag gag agt ctc atg agc ttg ttt gaa gag acc aag cgg aag     2448
Thr Ala Lys Glu Ser Leu Met Ser Leu Phe Glu Glu Thr Lys Arg Lys
                805                 810                 815

gca gaa cag atg gaa aag aag caa cat gag gaa ttt gac ctg agc cct     2496
Ala Glu Gln Met Glu Lys Lys Gln His Glu Glu Phe Asp Leu Ser Pro
            820                 825                 830

gac cag tgg cgg aaa gga aag aag tct act ttc cgg cat gtt gga gat     2544
Asp Gln Trp Arg Lys Gly Lys Lys Ser Thr Phe Arg His Val Gly Asp
        835                 840                 845

gac acc act cgc ctg gag atc tgg att cat cca tcc cat cca cgg tct     2592
Asp Thr Thr Arg Leu Glu Ile Trp Ile His Pro Ser His Pro Arg Ser
    850                 855                 860

tcc cag ggc act gag tct ggg aag gat tct gag caa gag aat ggg ctg     2640
Ser Gln Gly Thr Glu Ser Gly Lys Asp Ser Glu Gln Glu Asn Gly Leu

865                 870                 875                 880

ggc agc ctg agt ccc agt gat ctg aac aaa agg ttc atc ctc agt ttt     2688
Gly Ser Leu Ser Pro Ser Asp Leu Asn Lys Arg Phe Ile Leu Ser Phe
                885                 890                 895

ctc cat gcc cat ggg aag ctg ttt acc cgg att ggg atg gag aca ttc     2736
Leu His Ala His Gly Lys Leu Phe Thr Arg Ile Gly Met Glu Thr Phe
            900                 905                 910

cct gca gtg gct gag aag gtc ctc aag gag ttc cag gtg tta ctg cag     2784
Pro Ala Val Ala Glu Lys Val Leu Lys Glu Phe Gln Val Leu Leu Gln
        915                 920                 925

cac agc ccc tct ccc att gga agt acc cgc atg ctg cag ctt atg acc     2832
His Ser Pro Ser Pro Ile Gly Ser Thr Arg Met Leu Gln Leu Met Thr
    930                 935                 940

atc aat atg ttt gca gta cac aac tcc cag ctg aaa gac tgc ttc tcg     2880
Ile Asn Met Phe Ala Val His Asn Ser Gln Leu Lys Asp Cys Phe Ser
945                 950                 955                 960

gag gag tgc cgc tct gtg atc cag gaa caa gcc gca gct ctg ggc ttg     2928
Glu Glu Cys Arg Ser Val Ile Gln Glu Gln Ala Ala Ala Leu Gly Leu
                965                 970                 975

gcc atg ttt tct cta ctg gtc cgc cgc tgc acc tgc tta ctt aag gag     2976
Ala Met Phe Ser Leu Leu Val Arg Arg Cys Thr Cys Leu Leu Lys Glu
            980                 985                 990

tcc gcc aaa gct cag ctg tcc tct cct gag gac cag gat gac caa gac     3024
Ser Ala Lys Ala Gln Leu Ser Ser Pro Glu Asp Gln Asp Asp Gln Asp
        995                 1000                1005

gac atc aag gtg tct tcc ttt gtc ccg gac ctg aag gag ctg ctc ccc     3072
Asp Ile Lys Val Ser Ser Phe Val Pro Asp Leu Lys Glu Leu Leu Pro
    1010                1015                1020

agt gtc aaa gtc tgg tca gat tgg atg ctc ggc tac ccg gac acc tgg     3120
Ser Val Lys Val Trp Ser Asp Trp Met Leu Gly Tyr Pro Asp Thr Trp
1025                1030                1035                1040

aat cct cct ccc aca tcc ctg gat ctg ccc tcg cat gtt gct gtg gat     3168
Asn Pro Pro Pro Thr Ser Leu Asp Leu Pro Ser His Val Ala Val Asp
                1045                1050                1055

gta tgg tcg acg ctg gct gat ttc tgt aac ata ctg act gca gtg aat     3216
Val Trp Ser Thr Leu Ala Asp Phe Cys Asn Ile Leu Thr Ala Val Asn
            1060                1065                1070

cag tct gag gtg cca ctg tac aag gac ccg gat gat gac ctc acc ctt     3264
Gln Ser Glu Val Pro Leu Tyr Lys Asp Pro Asp Asp Asp Leu Thr Leu
        1075                1080                1085

ctt atc ctg gaa gag gat cgg ctt ctc tcg ggc ttt gtc ccc ttg ctg     3312
Leu Ile Leu Glu Glu Asp Arg Leu Leu Ser Gly Phe Val Pro Leu Leu
    1090                1095                1100

gct gcc cct cag gac ccc tgc tac gtg gag aaa acc tcg gat aag gtt     3360
Ala Ala Pro Gln Asp Pro Cys Tyr Val Glu Lys Thr Ser Asp Lys Val
1105                1110                1115                1120

att gca gct gac tgc aaa agg gtc aca gtg ctg aag tat ttt ctg gaa    3408
Ile Ala Ala Asp Cys Lys Arg Val Thr Val Leu Lys Tyr Phe Leu Glu
            1125                1130                1135

gcc ctt tgt gga caa gaa gag cct ctg ctg gca ttc aag ggt gga aag    3456
Ala Leu Cys Gly Gln Glu Glu Pro Leu Leu Ala Phe Lys Gly Gly Lys
        1140                1145                1150

tat gtg tca gtg gca ccc gtc cca gac acc atg gga aag gaa atg gga    3504
Tyr Val Ser Val Ala Pro Val Pro Asp Thr Met Gly Lys Glu Met Gly
    1155                1160                1165

agc caa gag gga aca cga ctg gaa gat gag gag gag gat gtg gtg att    3552
Ser Gln Glu Gly Thr Arg Leu Glu Asp Glu Glu Glu Asp Val Val Ile
1170                1175                1180

gaa gac ttt gag gaa gat tca gag gct gaa ggc agc gga ggc gag gat    3600
Glu Asp Phe Glu Glu Asp Ser Glu Ala Glu Gly Ser Gly Gly Glu Asp
1185                1190                1195                1200

gac atc agg gag ctt cgg gcc aag aag ctg gct ctg gcc agg aag ata    3648
Asp Ile Arg Glu Leu Arg Ala Lys Lys Leu Ala Leu Ala Arg Lys Ile
            1205                1210                1215

gct gag cag cag cgt cgc cag gaa aag atc cag gct gtc ctg gag gac    3696
Ala Glu Gln Gln Arg Arg Gln Glu Lys Ile Gln Ala Val Leu Glu Asp
        1220                1225                1230

cac agt cag atg agg cag atg gag ctc gaa atc aga cct ttg ttc ctc    3744
His Ser Gln Met Arg Gln Met Glu Leu Glu Ile Arg Pro Leu Phe Leu
    1235                1240                1245

gta cca gac acc aac ggc ttc att gac cac ctg gcc agt ctg gcg cgg    3792
Val Pro Asp Thr Asn Gly Phe Ile Asp His Leu Ala Ser Leu Ala Arg
1250                1255                1260

ctg ctg gag agc agg aag tac atc ctg gtg gtg ccc ctc atc gtg atc    3840
Leu Leu Glu Ser Arg Lys Tyr Ile Leu Val Val Pro Leu Ile Val Ile
1265                1270                1275                1280

aat gag ctg gac ggc ctg gcc aag ggg cag gag aca gac cac cgg gct    3888
Asn Glu Leu Asp Gly Leu Ala Lys Gly Gln Glu Thr Asp His Arg Ala
            1285                1290                1295

ggg ggc tac gcc cgt gtg gta caa gag aag gcc cgc aag tcc atc gag    3936
Gly Gly Tyr Ala Arg Val Val Gln Glu Lys Ala Arg Lys Ser Ile Glu
        1300                1305                1310

ttc ctc gag cag cga ttc gag agt cgg gac tct tgc ctg cga gcc ctg    3984
Phe Leu Glu Gln Arg Phe Glu Ser Arg Asp Ser Cys Leu Arg Ala Leu
    1315                1320                1325

acc agc cgt ggc aat gaa ctc gaa tcc atc gcc ttc cgc agt gag gac    4032
Thr Ser Arg Gly Asn Glu Leu Glu Ser Ile Ala Phe Arg Ser Glu Asp
1330                1335                1340

atc act ggc cag ctg ggt aac aac gat gat ctc atc ctg tcc tgc tgc    4080
Ile Thr Gly Gln Leu Gly Asn Asn Asp Asp Leu Ile Leu Ser Cys Cys
1345                1350                1355                1360

ctc cac tac tgc aaa gac aag gct aag gac ttc atg ccc gcc agc aaa     4128
Leu His Tyr Cys Lys Asp Lys Ala Lys Asp Phe Met Pro Ala Ser Lys
                1365                1370                1375

gag gag cca atc cgg cta ctg cgg gag gtg gtg ctg ttg acg gat gac     4176
Glu Glu Pro Ile Arg Leu Leu Arg Glu Val Val Leu Leu Thr Asp Asp
            1380                1385                1390

cgg aac ctg cgt gtg aag gcg ctc aca agg aat gtt cct gta cgg gac     4224
Arg Asn Leu Arg Val Lys Ala Leu Thr Arg Asn Val Pro Val Arg Asp
        1395                1400                1405

atc cca gcc ttc ctc acg tgg gcc cag gtg ggc tga                     4260
Ile Pro Ala Phe Leu Thr Trp Ala Gln Val Gly
    1410                1415                1420


<210> 2
<211> 1419
<212> PRT
<213> Homo sapiens

<400> 2
Met Ala Glu Gly Leu Glu Arg Val Arg Ile Ser Ala Ser Glu Leu Arg
1               5                   10                  15

Gly Ile Leu Ala Thr Leu Ala Pro Gln Ala Gly Ser Arg Glu Asn Met
            20                  25                  30

Lys Glu Leu Lys Glu Ala Arg Pro Arg Lys Asp Asn Arg Arg Pro Asp
        35                  40                  45

Leu Glu Ile Tyr Lys Pro Gly Leu Ser Arg Leu Arg Asn Lys Pro Lys
    50                  55                  60

Ile Lys Glu Pro Pro Gly Ser Glu Glu Phe Lys Asp Glu Ile Val Asn
65                  70                  75                  80

Asp Arg Asp Cys Ser Ala Val Glu Asn Gly Thr Gln Pro Val Lys Asp
                85                  90                  95

Val Cys Lys Glu Leu Asn Asn Gln Glu Gln Asn Gly Pro Ile Asp Pro
            100                 105                 110

Glu Asn Asn Arg Gly Gln Glu Ser Phe Pro Arg Thr Ala Gly Gln Glu
        115                 120                 125

Asp Arg Ser Leu Lys Ile Ile Lys Arg Thr Lys Lys Pro Asp Leu Gln
    130                 135                 140

Ile Tyr Gln Pro Gly Arg Arg Leu Gln Thr Val Ser Lys Glu Ser Ala
145                 150                 155                 160

Ser Arg Val Glu Glu Glu Glu Val Leu Asn Gln Val Glu Gln Leu Arg
                165                 170                 175

Val Glu Glu Asp Glu Cys Arg Gly Asn Val Ala Lys Glu Glu Val Ala
            180                 185                 190

Asn Lys Pro Asp Arg Ala Glu Ile Glu Lys Ser Pro Gly Gly Gly Arg
        195                 200                 205

Val Gly Ala Ala Lys Gly Glu Lys Gly Lys Arg Met Gly Lys Gly Glu
    210                 215                 220

Gly Val Arg Glu Thr His Asp Asp Pro Ala Arg Gly Arg Pro Gly Ser
225                 230                 235                 240

Ala Lys Arg Tyr Ser Arg Ser Asp Lys Arg Arg Asn Arg Tyr Arg Thr
                245                 250                 255

Arg Ser Thr Ser Ser Ala Gly Ser Asn Asn Ser Ala Glu Gly Ala Gly
            260                 265                 270

Leu Thr Asp Asn Gly Cys Arg Arg Arg Arg Gln Asp Arg Thr Lys Glu
        275                 280                 285

Arg Pro Arg Leu Lys Gln Gln Val Ser Val Ser Ser Thr Asp Ser Leu
    290                 295                 300

Asp Glu Asp Arg Ile Asp Glu Pro Asp Gly Leu Gly Pro Arg Arg Ser
305                 310                 315                 320

Ser Glu Arg Lys Arg His Leu Glu Arg Asn Trp Ser Gly Arg Gly Glu
                325                 330                 335

Gly Glu Gln Lys Thr Ser Ala Lys Glu Tyr Arg Gly Thr Leu Arg Val
            340                 345                 350

Thr Phe Asp Ala Glu Ala Met Asn Lys Glu Ser Pro Met Val Arg Ser
        355                 360                 365

Ala Arg Asp Asp Met Asp Arg Gly Lys Pro Asp Lys Gly Leu Ser Ser
    370                 375                 380

Gly Gly Lys Gly Ser Glu Lys Gln Glu Ser Lys Asn Pro Lys Gln Glu
385                 390                 395                 400

Leu Arg Gly Arg Gly Arg Gly Ile Leu Ile Leu Pro Ala His Thr Thr
                405                 410                 415

Leu Ser Val Asn Ser Ala Gly Ser Pro Glu Ser Ala Pro Leu Gly Pro
            420                 425                 430

Arg Leu Leu Phe Gly Ser Gly Ser Lys Gly Ser Arg Ser Trp Gly Arg
        435                 440                 445

Gly Gly Thr Thr Arg Arg Leu Trp Asp Pro Asn Asn Pro Asp Gln Lys
    450                 455                 460

Pro Ala Leu Lys Thr His Thr Pro Gln Leu His Phe Leu Asp Thr Asp
465                 470                 475                 480

Asp Glu Val Ser Pro Thr Ser Trp Gly Asp Ser Arg Gln Ala Gln Ala
                485                 490                 495

Ser Tyr Tyr Lys Phe Gln Asn Ser Asp Asn Pro Tyr Tyr Tyr Pro Arg
            500                 505                 510

Thr Pro Gly Pro Ala Ser Gln Tyr Pro Tyr Thr Gly Tyr Asn Pro Leu
        515                 520                 525

Gln Tyr Pro Val Gly Pro Thr Asn Gly Val Tyr Pro Gly Pro Tyr Tyr
530                 535                 540

Pro Gly Tyr Pro Thr Pro Ser Gly Gln Tyr Val Cys Ser Pro Leu Pro
545                 550                 555                 560

Thr Ser Thr Met Ser Pro Glu Glu Val Glu Gln His Met Arg Asn Leu
                565                 570                 575

Gln Gln Gln Glu Leu His Arg Leu Leu Arg Val Ala Asp Asn Gln Glu
            580                 585                 590

Leu Gln Leu Ser Asn Leu Leu Ser Arg Asp Arg Ile Ser Pro Glu Gly
        595                 600                 605

Leu Glu Lys Met Ala Gln Leu Arg Ala Glu Leu Leu Gln Leu Tyr Glu
    610                 615                 620

Arg Cys Ile Leu Leu Asp Ile Glu Phe Ser Asp Asn Gln Asn Val Asp
625                 630                 635                 640

Gln Ile Leu Trp Lys Asn Ala Phe Tyr Gln Val Ile Glu Lys Phe Arg
                645                 650                 655

Gln Leu Val Lys Asp Pro Asn Val Glu Asn Pro Glu Gln Ile Arg Asn
            660                 665                 670

Arg Leu Leu Glu Leu Leu Asp Glu Gly Ser Asp Phe Phe Asp Ser Leu
        675                 680                 685

Leu Gln Lys Leu Gln Val Thr Tyr Lys Phe Lys Leu Glu Asp Tyr Met
    690                 695                 700

Asp Gly Leu Ala Ile Arg Ser Lys Pro Leu Arg Lys Thr Val Lys Tyr
705                 710                 715                 720

Ala Leu Ile Ser Ala Gln Arg Cys Met Ile Cys Gln Gly Asp Ile Ala
                725                 730                 735

Arg Tyr Arg Glu Gln Ala Ser Asp Thr Ala Asn Tyr Gly Lys Ala Arg
            740                 745                 750

Ser Trp Tyr Leu Lys Ala Gln His Ile Ala Pro Lys Asn Gly Arg Pro
        755                 760                 765

Tyr Asn Gln Leu Ala Leu Leu Ala Val Tyr Thr Arg Arg Lys Leu Asp
    770                 775                 780

Ala Val Tyr Tyr Tyr Met Arg Ser Leu Ala Ala Ser Asn Pro Ile Leu
785                 790                 795                 800

Thr Ala Lys Glu Ser Leu Met Ser Leu Phe Glu Glu Thr Lys Arg Lys
                805                 810                 815

Ala Glu Gln Met Glu Lys Lys Gln His Glu Glu Phe Asp Leu Ser Pro
            820                 825                 830

Asp Gln Trp Arg Lys Gly Lys Lys Ser Thr Phe Arg His Val Gly Asp
        835                 840                 845

Asp Thr Thr Arg Leu Glu Ile Trp Ile His Pro Ser His Pro Arg Ser

850                 855                 860

Ser Gln Gly Thr Glu Ser Gly Lys Asp Ser Glu Gln Glu Asn Gly Leu
865                 870                 875                 880

Gly Ser Leu Ser Pro Ser Asp Leu Asn Lys Arg Phe Ile Leu Ser Phe
                885                 890                 895

Leu His Ala His Gly Lys Leu Phe Thr Arg Ile Gly Met Glu Thr Phe
            900                 905                 910

Pro Ala Val Ala Glu Lys Val Leu Lys Glu Phe Gln Val Leu Leu Gln
        915                 920                 925

His Ser Pro Ser Pro Ile Gly Ser Thr Arg Met Leu Gln Leu Met Thr
    930                 935                 940

Ile Asn Met Phe Ala Val His Asn Ser Gln Leu Lys Asp Cys Phe Ser
945                 950                 955                 960

Glu Glu Cys Arg Ser Val Ile Gln Glu Gln Ala Ala Ala Leu Gly Leu
                965                 970                 975

Ala Met Phe Ser Leu Leu Val Arg Arg Cys Thr Cys Leu Leu Lys Glu
            980                 985                 990

Ser Ala Lys Ala Gln Leu Ser Ser Pro Glu Asp Gln Asp Asp Gln Asp
        995                 1000                1005

Asp Ile Lys Val Ser Ser Phe Val Pro Asp Leu Lys Glu Leu Leu Pro
   1010                1015                1020

Ser Val Lys Val Trp Ser Asp Trp Met Leu Gly Tyr Pro Asp Thr Trp
025                 1030                1035                1040

Asn Pro Pro Pro Thr Ser Leu Asp Leu Pro Ser His Val Ala Val Asp
               1045                1050                1055

Val Trp Ser Thr Leu Ala Asp Phe Cys Asn Ile Leu Thr Ala Val Asn
           1060                1065                1070

Gln Ser Glu Val Pro Leu Tyr Lys Asp Pro Asp Asp Asp Leu Thr Leu
       1075                1080                1085

Leu Ile Leu Glu Glu Asp Arg Leu Leu Ser Gly Phe Val Pro Leu Leu
   1090                1095                1100

Ala Ala Pro Gln Asp Pro Cys Tyr Val Glu Lys Thr Ser Asp Lys Val
105                 1110                1115                1120

Ile Ala Ala Asp Cys Lys Arg Val Thr Val Leu Lys Tyr Phe Leu Glu
               1125                1130                1135

Ala Leu Cys Gly Gln Glu Glu Pro Leu Leu Ala Phe Lys Gly Gly Lys
           1140                1145                1150

Tyr Val Ser Val Ala Pro Val Pro Asp Thr Met Gly Lys Glu Met Gly
       1155                1160                1165

Ser Gln Glu Gly Thr Arg Leu Glu Asp Glu Glu Glu Asp Val Val Ile
   1170                1175                1180

Glu Asp Phe Glu Glu Asp Ser Glu Ala Glu Gly Ser Gly Gly Glu Asp
185                 1190                1195                1200

Asp Ile Arg Glu Leu Arg Ala Lys Lys Leu Ala Leu Ala Arg Lys Ile
                1205                1210                1215

Ala Glu Gln Gln Arg Arg Gln Glu Lys Ile Gln Ala Val Leu Glu Asp
            1220                1225                1230

His Ser Gln Met Arg Gln Met Glu Leu Glu Ile Arg Pro Leu Phe Leu
        1235                1240                1245

Val Pro Asp Thr Asn Gly Phe Ile Asp His Leu Ala Ser Leu Ala Arg
    1250                1255                1260

Leu Leu Glu Ser Arg Lys Tyr Ile Leu Val Val Pro Leu Ile Val Ile
265                 1270                1275                1280

Asn Glu Leu Asp Gly Leu Ala Lys Gly Gln Glu Thr Asp His Arg Ala
                1285                1290                1295

Gly Gly Tyr Ala Arg Val Val Gln Glu Lys Ala Arg Lys Ser Ile Glu
            1300                1305                1310

Phe Leu Glu Gln Arg Phe Glu Ser Arg Asp Ser Cys Leu Arg Ala Leu
        1315                1320                1325

Thr Ser Arg Gly Asn Glu Leu Glu Ser Ile Ala Phe Arg Ser Glu Asp
    1330                1335                1340

Ile Thr Gly Gln Leu Gly Asn Asn Asp Asp Leu Ile Leu Ser Cys Cys
345                 1350                1355                1360

Leu His Tyr Cys Lys Asp Lys Ala Lys Asp Phe Met Pro Ala Ser Lys
                1365                1370                1375

Glu Glu Pro Ile Arg Leu Leu Arg Glu Val Val Leu Leu Thr Asp Asp
            1380                1385                1390

Arg Asn Leu Arg Val Lys Ala Leu Thr Arg Asn Val Pro Val Arg Asp
        1395                1400                1405

Ile Pro Ala Phe Leu Thr Trp Ala Gln Val Gly
    1410                1415

<210> 3
<211> 3051
<212> DNA
<213> Homo sapiens

<220>
<221> CDS
<222> (1)..(3051)

<400> 3
atg agc caa ggc ccc cct aca ggg gag agc agc gag ccc gaa gca aaa      48
Met Ser Gln Gly Pro Pro Thr Gly Glu Ser Ser Glu Pro Glu Ala Lys
1               5                   10                  15

gtc ctc cac act aag cgg ctt tac cgg gct gtg gtg gag gct gtg cat     96
Val Leu His Thr Lys Arg Leu Tyr Arg Ala Val Val Glu Ala Val His
                20                  25                  30

cga ctt gac ctc atc ctt tgc aac aaa act gct tat caa gaa gta ttc     144
Arg Leu Asp Leu Ile Leu Cys Asn Lys Thr Ala Tyr Gln Glu Val Phe
            35                  40                  45

aaa cca gaa aac att agc ctg agg aac aag ctg cgt gag ctc tgc gtc     192
Lys Pro Glu Asn Ile Ser Leu Arg Asn Lys Leu Arg Glu Leu Cys Val
    50                  55                  60

aag ctt atg ttc ctg cac cca gtg gac tat ggg aga aag gct gag gag     240
Lys Leu Met Phe Leu His Pro Val Asp Tyr Gly Arg Lys Ala Glu Glu
65                  70                  75                  80

ctg ctg tgg aga aag gta tac tat gaa gtt atc cag ctt atc aag act     288
Leu Leu Trp Arg Lys Val Tyr Tyr Glu Val Ile Gln Leu Ile Lys Thr
                85                  90                  95

aac aaa aag cac atc cac agc cgg agc act ttg gaa tgt gcc tac agg     336
Asn Lys Lys His Ile His Ser Arg Ser Thr Leu Glu Cys Ala Tyr Arg
            100                 105                 110

acg cac ctg gtt gct ggt att ggc ttc tac cag cat ctc ctt ctc tat     384
Thr His Leu Val Ala Gly Ile Gly Phe Tyr Gln His Leu Leu Leu Tyr
        115                 120                 125

atc cag tcc cac tac cag ctg gaa ctg cag tgc tgc atc gac tgg acc     432
Ile Gln Ser His Tyr Gln Leu Glu Leu Gln Cys Cys Ile Asp Trp Thr
    130                 135                 140

cat gtc act gac ccc ctc ata gga tgc aag aag cca gtg tct gcc tca     480
His Val Thr Asp Pro Leu Ile Gly Cys Lys Lys Pro Val Ser Ala Ser
145                 150                 155                 160

ggg aag gag atg gat tgg gca cag atg gca tgt cac cga tgt ctg gtg     528
Gly Lys Glu Met Asp Trp Ala Gln Met Ala Cys His Arg Cys Leu Val
                165                 170                 175

tat ctg ggg gat ttg tcc cga tat cag aat gaa tta gct ggc gta gat     576
Tyr Leu Gly Asp Leu Ser Arg Tyr Gln Asn Glu Leu Ala Gly Val Asp
            180                 185                 190

acc gag ctg cta gcc gag aga ttt tac tac caa gcc ctg tca gta gct     624
Thr Glu Leu Leu Ala Glu Arg Phe Tyr Tyr Gln Ala Leu Ser Val Ala
        195                 200                 205

cct cag att gga atg ccc ttc aat cag ctg ggc acc ctg gca ggc agc     672
Pro Gln Ile Gly Met Pro Phe Asn Gln Leu Gly Thr Leu Ala Gly Ser
    210                 215                 220

aag tac tat aat gtg gaa gcc atg tat tgc tac ctg cgc tgc atc cag     720
Lys Tyr Tyr Asn Val Glu Ala Met Tyr Cys Tyr Leu Arg Cys Ile Gln
225                 230                 235                 240

tca gaa gtg tcc ttt gag gga gcc tat ggg aac ctc aag cgg ctg tat     768
Ser Glu Val Ser Phe Glu Gly Ala Tyr Gly Asn Leu Lys Arg Leu Tyr
                245                 250                 255

gac aag gca gcc aaa atg tac cac caa ctg aag aag tgt gag act cgg     816
Asp Lys Ala Ala Lys Met Tyr His Gln Leu Lys Lys Cys Glu Thr Arg
            260                 265                 270

aaa ctg tct cct ggc aaa aag cga tgt aaa gac att aaa agg ttg cta     864
Lys Leu Ser Pro Gly Lys Lys Arg Cys Lys Asp Ile Lys Arg Leu Leu
        275                 280                 285

gtg aac ttt atg tat ctg caa agc ctc cta cag ccc aaa agc agc tcc     912
Val Asn Phe Met Tyr Leu Gln Ser Leu Leu Gln Pro Lys Ser Ser Ser
    290                 295                 300

gtg gac tca gag ctg acc tca ctt tgc cag tca gtc ctg gag gac ttc     960
Val Asp Ser Glu Leu Thr Ser Leu Cys Gln Ser Val Leu Glu Asp Phe
305                 310                 315                 320

aac ctc tgc ctc ttc tac ctg ccc tcc tca ccc aac ctc agc ctg gcc     1008
Asn Leu Cys Leu Phe Tyr Leu Pro Ser Ser Pro Asn Leu Ser Leu Ala
                325                 330                 335

agt gag gat gag gag gag tat gag agt gga tat gct ttc ctc ccg gac     1056
Ser Glu Asp Glu Glu Glu Tyr Glu Ser Gly Tyr Ala Phe Leu Pro Asp
            340                 345                 350

ctt ctc atc ttt caa atg gtc atc atc tgc ctt atg tgt gtg cac agc     1104
Leu Leu Ile Phe Gln Met Val Ile Ile Cys Leu Met Cys Val His Ser
        355                 360                 365

ttg gag aga gca gga tcc aag cag tac agt gca gcc att gcc ttc acc     1152
Leu Glu Arg Ala Gly Ser Lys Gln Tyr Ser Ala Ala Ile Ala Phe Thr
    370                 375                 380

ctg gcc ctc ttt tcc cac ctc gtc aat cat gtc aac ata cgg ctg cag     1200
Leu Ala Leu Phe Ser His Leu Val Asn His Val Asn Ile Arg Leu Gln
385                 390                 395                 400

gct gag ctg gaa gag ggc gag aat ccc gtc ccg gca ttc cag agt gat     1248
Ala Glu Leu Glu Glu Gly Glu Asn Pro Val Pro Ala Phe Gln Ser Asp
                405                 410                 415

ggc aca gat gaa cca gag tcc aag gaa cct gtg gag aaa gag gag gag     1296
Gly Thr Asp Glu Pro Glu Ser Lys Glu Pro Val Glu Lys Glu Glu Glu
            420                 425                 430

cca gat cct gag cct cct cct gta aca ccc caa gtg ggt gag ggc aga     1344
Pro Asp Pro Glu Pro Pro Pro Val Thr Pro Gln Val Gly Glu Gly Arg
        435                 440                 445

aag agc cgt aag ttc tct cgc ctc tcc tgt ctc cgc cgt cgc cgc cac     1392
Lys Ser Arg Lys Phe Ser Arg Leu Ser Cys Leu Arg Arg Arg Arg His
    450                 455                 460

cca ccc aaa gtt ggt gat gac agt gac ctg agt gaa ggc ttt gaa tcg     1440
Pro Pro Lys Val Gly Asp Asp Ser Asp Leu Ser Glu Gly Phe Glu Ser
465                 470                 475                 480

gac tca agc cat gac tca gcc cgg gcc agt gag ggc tca gac agt ggc     1488
Asp Ser Ser His Asp Ser Ala Arg Ala Ser Glu Gly Ser Asp Ser Gly
                485                 490                 495

tct gac aag agt ctt gaa ggt ggg gga acg gcc ttt gat gct gaa aca     1536

Ser Asp Lys Ser Leu Glu Gly Gly Gly Thr Ala Phe Asp Ala Glu Thr
            500                 505                 510

gac tcg gaa atg aat agc cag gag tcc cga tca gac ttg gaa gat atg      1584
Asp Ser Glu Met Asn Ser Gln Glu Ser Arg Ser Asp Leu Glu Asp Met
        515                 520                 525

gag gaa gag gag ggg aca cgg tca cca acc ctg gag ccc cct cgg ggc      1632
Glu Glu Glu Glu Gly Thr Arg Ser Pro Thr Leu Glu Pro Pro Arg Gly
    530                 535                 540

aga tca gag gct ccc gat tcc ctc aat ggc cca ctg ggc ccc agt gag      1680
Arg Ser Glu Ala Pro Asp Ser Leu Asn Gly Pro Leu Gly Pro Ser Glu
545                 550                 555                 560

gct agc att gcc agc aat cta caa gcc atg tcc acc cag atg ttc cag      1728
Ala Ser Ile Ala Ser Asn Leu Gln Ala Met Ser Thr Gln Met Phe Gln
                565                 570                 575

act aag cgc tgc ttc cga ctg gcc ccc acc ttt agc aac ctg ctc ctc      1776
Thr Lys Arg Cys Phe Arg Leu Ala Pro Thr Phe Ser Asn Leu Leu Leu
            580                 585                 590

cag ccc acc acc aac cct cat acc tcg gcc agc cac agg cct tgc gtc      1824
Gln Pro Thr Thr Asn Pro His Thr Ser Ala Ser His Arg Pro Cys Val
        595                 600                 605

aat ggg gat gta gac aag cct tca gag cca gcc tct gag gag ggc tct      1872
Asn Gly Asp Val Asp Lys Pro Ser Glu Pro Ala Ser Glu Glu Gly Ser
    610                 615                 620

gag tcg gag ggg agt gag tcc agt gga cgc tcc tgt cgg aat gag cgc      1920
Glu Ser Glu Gly Ser Glu Ser Ser Gly Arg Ser Cys Arg Asn Glu Arg
625                 630                 635                 640

agc atc cag gag aag ctt cag gtc ctg atg gcc gaa ggt ctg ctt cct      1968
Ser Ile Gln Glu Lys Leu Gln Val Leu Met Ala Glu Gly Leu Leu Pro
                645                 650                 655

gct gtg aaa gtc ttc ctg gac tgg ctt cgg acc aac ccc gac ctc atc      2016
Ala Val Lys Val Phe Leu Asp Trp Leu Arg Thr Asn Pro Asp Leu Ile
            660                 665                 670

atc gtg tgt gca cag agc tct caa agt ctg tgg aac cgc ctg tct gtg      2064
Ile Val Cys Ala Gln Ser Ser Gln Ser Leu Trp Asn Arg Leu Ser Val
        675                 680                 685

ttg ctg aat ctg ttg cct gct gct ggt gaa ctc cag gag tct ggc ctg      2112
Leu Leu Asn Leu Leu Pro Ala Ala Gly Glu Leu Gln Glu Ser Gly Leu
    690                 695                 700

gcc ttg tgt cct gag gtc caa gat ctt ctt gaa ggt tgt gaa ctg cct      2160
Ala Leu Cys Pro Glu Val Gln Asp Leu Leu Glu Gly Cys Glu Leu Pro
705                 710                 715                 720

gac ctc ccc tct agc ctt ctg ctc cca gag gac atg gct ctt cgt aac      2208
Asp Leu Pro Ser Ser Leu Leu Leu Pro Glu Asp Met Ala Leu Arg Asn
                725                 730                 735

ctg ccc ccg ctc cga gct gcc cac aga cgc ttt aac ttt gac acg gat      2256
Leu Pro Pro Leu Arg Ala Ala His Arg Arg Phe Asn Phe Asp Thr Asp

            740                 745                 750

cgg ccc ctg ctc agc acc tta gag gag tca gtg gtg cgc atc tgc tgc 2304
Arg Pro Leu Leu Ser Thr Leu Glu Glu Ser Val Val Arg Ile Cys Cys
        755                 760                 765

atc cgc agc ttt ggt cat ttc atc gcc cgc ctg caa ggc agc atc ctg 2352
Ile Arg Ser Phe Gly His Phe Ile Ala Arg Leu Gln Gly Ser Ile Leu
    770                 775                 780

cag ttc aac cca gag gtt ggc atc ttc gtc agc att gcc cag tct gag 2400
Gln Phe Asn Pro Glu Val Gly Ile Phe Val Ser Ile Ala Gln Ser Glu
785                 790                 795                 800

cag gag agc ctg ctg cag cag gcc cag gca cag ttc cga atg gca cag 2448
Gln Glu Ser Leu Leu Gln Gln Ala Gln Ala Gln Phe Arg Met Ala Gln
                805                 810                 815

gag gaa gct cgt cgg aac agg ctc atg aga gac atg gct cag cta cga 2496
Glu Glu Ala Arg Arg Asn Arg Leu Met Arg Asp Met Ala Gln Leu Arg
            820                 825                 830

ctt cag ctc gaa gtg tct cag ctg gag ggc agc ctg cag cag ccc aag 2544
Leu Gln Leu Glu Val Ser Gln Leu Glu Gly Ser Leu Gln Gln Pro Lys
        835                 840                 845

gcc cag tca gcc atg tct ccc tac ctc gtc cct gac acc cag gcc ctc 2592
Ala Gln Ser Ala Met Ser Pro Tyr Leu Val Pro Asp Thr Gln Ala Leu
    850                 855                 860

tgc cac cat ctc cct gtc atc cgc caa ctg gcc acc agt ggc cgc ttc 2640
Cys His His Leu Pro Val Ile Arg Gln Leu Ala Thr Ser Gly Arg Phe
865                 870                 875                 880

att gtc atc atc cca agg aca gtg atc gat ggc ctg gat ttg ctg aag 2688
Ile Val Ile Ile Pro Arg Thr Val Ile Asp Gly Leu Asp Leu Leu Lys
                885                 890                 895

aag gaa cac cca ggg gcc cgg gat ggg att cgg tac ctg gag gca gag 2736
Lys Glu His Pro Gly Ala Arg Asp Gly Ile Arg Tyr Leu Glu Ala Glu
            900                 905                 910

ttt aaa aaa gga aac agg tac att cgc tgc cag aaa gag gtg gga aag 2784
Phe Lys Lys Gly Asn Arg Tyr Ile Arg Cys Gln Lys Glu Val Gly Lys
        915                 920                 925

agc ttt gag cgg cat aag ctg aag agg cag gat gca gat gcc tgg act 2832
Ser Phe Glu Arg His Lys Leu Lys Arg Gln Asp Ala Asp Ala Trp Thr
    930                 935                 940

ctc tat aag atc cta gac agc tgc aaa cag ctg act ctg gcc cag ggg 2880
Leu Tyr Lys Ile Leu Asp Ser Cys Lys Gln Leu Thr Leu Ala Gln Gly
945                 950                 955                 960

gca ggt gag gag gat ccg agt ggc atg gtg acc atc atc aca ggc ctt 2928
Ala Gly Glu Glu Asp Pro Ser Gly Met Val Thr Ile Ile Thr Gly Leu
                965                 970                 975

cca ctg gac aac ccc agc gtg ctt tca ggc ccc atg cag gca gcc ctg 2976
Pro Leu Asp Asn Pro Ser Val Leu Ser Gly Pro Met Gln Ala Ala Leu
            980                 985                 990

cag gcc gct gcc cac gcc agt gtg gac atc aag aat gtt ctg gac ttc     3024
Gln Ala Ala Ala His Ala Ser Val Asp Ile Lys Asn Val Leu Asp Phe
        995                 1000                1005

tac aag cag tgg aag gaa att ggt tga                                 3051
Tyr Lys Gln Trp Lys Glu Ile Gly
    1010                1015

<210> 4
<211> 1016
<212> PRT
<213> Homo sapiens

<400> 4
Met Ser Gln Gly Pro Pro Thr Gly Glu Ser Ser Glu Pro Glu Ala Lys
1               5                   10                  15

Val Leu His Thr Lys Arg Leu Tyr Arg Ala Val Val Glu Ala Val His
            20                  25                  30

Arg Leu Asp Leu Ile Leu Cys Asn Lys Thr Ala Tyr Gln Glu Val Phe
        35                  40                  45

Lys Pro Glu Asn Ile Ser Leu Arg Asn Lys Leu Arg Glu Leu Cys Val
    50                  55                  60

Lys Leu Met Phe Leu His Pro Val Asp Tyr Gly Arg Lys Ala Glu Glu
65                  70                  75                  80

Leu Leu Trp Arg Lys Val Tyr Tyr Glu Val Ile Gln Leu Ile Lys Thr
                85                  90                  95

Asn Lys Lys His Ile His Ser Arg Ser Thr Leu Glu Cys Ala Tyr Arg
            100                 105                 110

Thr His Leu Val Ala Gly Ile Gly Phe Tyr Gln His Leu Leu Leu Tyr
        115                 120                 125

Ile Gln Ser His Tyr Gln Leu Glu Leu Gln Cys Cys Ile Asp Trp Thr
    130                 135                 140

His Val Thr Asp Pro Leu Ile Gly Cys Lys Lys Pro Val Ser Ala Ser
145                 150                 155                 160

Gly Lys Glu Met Asp Trp Ala Gln Met Ala Cys His Arg Cys Leu Val
                165                 170                 175

Tyr Leu Gly Asp Leu Ser Arg Tyr Gln Asn Glu Leu Ala Gly Val Asp
            180                 185                 190

Thr Glu Leu Leu Ala Glu Arg Phe Tyr Tyr Gln Ala Leu Ser Val Ala
        195                 200                 205

Pro Gln Ile Gly Met Pro Phe Asn Gln Leu Gly Thr Leu Ala Gly Ser
    210                 215                 220

Lys Tyr Tyr Asn Val Glu Ala Met Tyr Cys Tyr Leu Arg Cys Ile Gln
225                 230                 235                 240

Ser Glu Val Ser Phe Glu Gly Ala Tyr Gly Asn Leu Lys Arg Leu Tyr
                245                 250                 255

Asp Lys Ala Ala Lys Met Tyr His Gln Leu Lys Lys Cys Glu Thr Arg
            260                 265                 270

Lys Leu Ser Pro Gly Lys Lys Arg Cys Lys Asp Ile Lys Arg Leu Leu
        275                 280                 285

Val Asn Phe Met Tyr Leu Gln Ser Leu Leu Gln Pro Lys Ser Ser Ser
    290                 295                 300

Val Asp Ser Glu Leu Thr Ser Leu Cys Gln Ser Val Leu Glu Asp Phe
305                 310                 315                 320

Asn Leu Cys Leu Phe Tyr Leu Pro Ser Ser Pro Asn Leu Ser Leu Ala
                325                 330                 335

Ser Glu Asp Glu Glu Glu Tyr Glu Ser Gly Tyr Ala Phe Leu Pro Asp
            340                 345                 350

Leu Leu Ile Phe Gln Met Val Ile Ile Cys Leu Met Cys Val His Ser
        355                 360                 365

Leu Glu Arg Ala Gly Ser Lys Gln Tyr Ser Ala Ala Ile Ala Phe Thr
    370                 375                 380

Leu Ala Leu Phe Ser His Leu Val Asn His Val Asn Ile Arg Leu Gln
385                 390                 395                 400

Ala Glu Leu Glu Glu Gly Glu Asn Pro Val Pro Ala Phe Gln Ser Asp
                405                 410                 415

Gly Thr Asp Glu Pro Glu Ser Lys Glu Pro Val Glu Lys Glu Glu Glu
            420                 425                 430

Pro Asp Pro Glu Pro Pro Pro Val Thr Pro Gln Val Gly Glu Gly Arg
        435                 440                 445

Lys Ser Arg Lys Phe Ser Arg Leu Ser Cys Leu Arg Arg Arg Arg His
    450                 455                 460

Pro Pro Lys Val Gly Asp Asp Ser Asp Leu Ser Glu Gly Phe Glu Ser
465                 470                 475                 480

Asp Ser Ser His Asp Ser Ala Arg Ala Ser Glu Gly Ser Asp Ser Gly
                485                 490                 495

Ser Asp Lys Ser Leu Glu Gly Gly Gly Thr Ala Phe Asp Ala Glu Thr
            500                 505                 510

Asp Ser Glu Met Asn Ser Gln Glu Ser Arg Ser Asp Leu Glu Asp Met
        515                 520                 525

Glu Glu Glu Glu Gly Thr Arg Ser Pro Thr Leu Glu Pro Pro Arg Gly
    530                 535                 540

Arg Ser Glu Ala Pro Asp Ser Leu Asn Gly Pro Leu Gly Pro Ser Glu
545                 550                 555                 560

Ala Ser Ile Ala Ser Asn Leu Gln Ala Met Ser Thr Gln Met Phe Gln

                565                 570                 575

Thr Lys Arg Cys Phe Arg Leu Ala Pro Thr Phe Ser Asn Leu Leu Leu
            580                 585                 590

Gln Pro Thr Thr Asn Pro His Thr Ser Ala Ser His Arg Pro Cys Val
        595                 600                 605

Asn Gly Asp Val Asp Lys Pro Ser Glu Pro Ala Ser Glu Glu Gly Ser
    610                 615                 620

Glu Ser Glu Gly Ser Glu Ser Ser Gly Arg Ser Cys Arg Asn Glu Arg
625                 630                 635                 640

Ser Ile Gln Glu Lys Leu Gln Val Leu Met Ala Glu Gly Leu Leu Pro
                645                 650                 655

Ala Val Lys Val Phe Leu Asp Trp Leu Arg Thr Asn Pro Asp Leu Ile
            660                 665                 670

Ile Val Cys Ala Gln Ser Ser Gln Ser Leu Trp Asn Arg Leu Ser Val
        675                 680                 685

Leu Leu Asn Leu Leu Pro Ala Ala Gly Glu Leu Gln Glu Ser Gly Leu
    690                 695                 700

Ala Leu Cys Pro Glu Val Gln Asp Leu Leu Glu Gly Cys Glu Leu Pro
705                 710                 715                 720

Asp Leu Pro Ser Ser Leu Leu Leu Pro Glu Asp Met Ala Leu Arg Asn
                725                 730                 735

Leu Pro Pro Leu Arg Ala Ala His Arg Arg Phe Asn Phe Asp Thr Asp
            740                 745                 750

Arg Pro Leu Leu Ser Thr Leu Glu Glu Ser Val Val Arg Ile Cys Cys
        755                 760                 765

Ile Arg Ser Phe Gly His Phe Ile Ala Arg Leu Gln Gly Ser Ile Leu
    770                 775                 780

Gln Phe Asn Pro Glu Val Gly Ile Phe Val Ser Ile Ala Gln Ser Glu
785                 790                 795                 800

Gln Glu Ser Leu Leu Gln Gln Ala Gln Ala Gln Phe Arg Met Ala Gln
                805                 810                 815

Glu Glu Ala Arg Arg Asn Arg Leu Met Arg Asp Met Ala Gln Leu Arg
            820                 825                 830

Leu Gln Leu Glu Val Ser Gln Leu Glu Gly Ser Leu Gln Gln Pro Lys
        835                 840                 845

Ala Gln Ser Ala Met Ser Pro Tyr Leu Val Pro Asp Thr Gln Ala Leu
    850                 855                 860

Cys His His Leu Pro Val Ile Arg Gln Leu Ala Thr Ser Gly Arg Phe
865                 870                 875                 880

Ile Val Ile Ile Pro Arg Thr Val Ile Asp Gly Leu Asp Leu Leu Lys
                885                 890                 895

Lys Glu His Pro Gly Ala Arg Asp Gly Ile Arg Tyr Leu Glu Ala Glu
            900                 905                 910

Phe Lys Lys Gly Asn Arg Tyr Ile Arg Cys Gln Lys Glu Val Gly Lys
        915                 920                 925

Ser Phe Glu Arg His Lys Leu Lys Arg Gln Asp Ala Asp Ala Trp Thr
    930                 935                 940

Leu Tyr Lys Ile Leu Asp Ser Cys Lys Gln Leu Thr Leu Ala Gln Gly
945                 950                 955                 960

Ala Gly Glu Glu Asp Pro Ser Gly Met Val Thr Ile Ile Thr Gly Leu
                965                 970                 975

Pro Leu Asp Asn Pro Ser Val Leu Ser Gly Pro Met Gln Ala Ala Leu
            980                 985                 990

Gln Ala Ala Ala His Ala Ser Val Asp Ile Lys Asn Val Leu Asp Phe
        995                 1000                1005

Tyr Lys Gln Trp Lys Glu Ile Gly
    1010                1015

<210> 5
<211> 1265
<212> PRT
<213> Homo sapiens

<400> 5
Xaa Cys Leu Lys Pro Phe Tyr Leu Met Arg Asn Trp Met Phe Leu Gln
1               5                   10                  15

Ile Val Ile Val Gly Glu Asn Val Ser Phe Lys Ser Gln Met Arg Thr
            20                  25                  30

Glu Asn Leu Lys Ser Glu Glu His Leu Lys Ser Ser Asn Ile Arg Gln
        35                  40                  45

Ala Glu Val Leu Lys Ala Asp Met Thr Asp Ser Lys Leu Gly Pro Ala
    50                  55                  60

Glu Val Trp Thr Ser Arg Gln Ala Leu Gln Asp Leu Tyr Gln Lys Met
65                  70                  75                  80

Leu Val Thr Asp Leu Glu Tyr Ala Leu Asp Lys Lys Val Glu Gln Asp
                85                  90                  95

Leu Trp Asn His Ala Phe Lys Asn Gln Ile Thr Thr Leu Gln Gly Gln
            100                 105                 110

Ala Lys Asn Arg Ala Asn Pro Asn Arg Ser Glu Val Gln Ala Asn Leu
        115                 120                 125

Ser Leu Phe Leu Glu Ala Ala Ser Gly Phe Tyr Thr Gln Leu Leu Gln
    130                 135                 140

Glu Leu Cys Thr Val Phe Asn Val Asp Leu Pro Cys Arg Val Lys Ser

145                 150                 155                 160

Ser Gln Leu Gly Ile Ile Ser Asn Lys Gln Thr His Thr Ser Ala Ile
                165                 170                 175

Val Lys Pro Gln Ser Ser Ser Cys Ser Tyr Ile Cys Gln His Cys Leu
            180                 185                 190

Val His Leu Gly Asp Ile Ala Arg Tyr Arg Asn Gln Thr Ser Gln Ala
        195                 200                 205

Glu Ser Tyr Tyr Arg His Ala Ala Gln Leu Val Pro Ser Asn Gly Gln
    210                 215                 220

Pro Tyr Asn Gln Leu Ala Ile Leu Ala Ser Ser Lys Gly Asp His Leu
225                 230                 235                 240

Thr Thr Ile Phe Tyr Tyr Cys Arg Ser Ile Ala Val Lys Phe Pro Phe
                245                 250                 255

Pro Ala Ala Ser Thr Asn Leu Gln Lys Ala Leu Ser Lys Ala Leu Glu
            260                 265                 270

Ser Arg Asp Glu Val Lys Thr Lys Trp Gly Val Ser Asp Phe Ile Lys
        275                 280                 285

Ala Phe Ile Lys Phe His Gly His Val Tyr Leu Ser Lys Ser Leu Glu
    290                 295                 300

Lys Leu Ser Pro Leu Arg Glu Lys Leu Glu Glu Gln Phe Lys Arg Leu
305                 310                 315                 320

Leu Phe Gln Lys Ala Phe Asn Ser Gln Gln Leu Val His Val Thr Val
                325                 330                 335

Ile Asn Leu Phe Gln Leu His His Leu Arg Asp Phe Ser Asn Glu Thr
            340                 345                 350

Glu Gln His Thr Tyr Ser Gln Asp Glu Gln Leu Cys Trp Thr Gln Leu
        355                 360                 365

Leu Ala Leu Phe Met Ser Phe Leu Gly Ile Leu Cys Lys Cys Pro Leu
    370                 375                 380

Gln Asn Glu Ser Gln Glu Glu Ser Tyr Asn Ala Tyr Pro Leu Pro Ala
385                 390                 395                 400

Val Lys Val Ser Met Asp Trp Leu Arg Leu Arg Pro Arg Val Phe Gln
                405                 410                 415

Glu Ala Val Val Asp Glu Arg Gln Tyr Ile Trp Pro Trp Leu Ile Ser
            420                 425                 430

Leu Leu Asn Ser Phe His Pro His Glu Glu Asp Leu Ser Ser Ile Ser
        435                 440                 445

Gly His Gln Gly Ile Thr Gly Asp Lys Glu Gly Gln Gln Arg Arg Ile
    450                 455                 460

Arg Gln Gln Arg Leu Ile Ser Ile Gly Lys Trp Ile Ala Asp Asn Gln
465                 470                 475                 480

Pro Arg Leu Ile Gln Cys Glu Asn Glu Val Gly Lys Leu Leu Phe Ile
                485                 490                 495

Thr Glu Ile Pro Glu Leu Ile Leu Glu Asp Pro Ser Glu Ala Lys Glu
            500                 505                 510

Asn Leu Ile Leu Gln Glu Thr Ser Val Ile Glu Ser Leu Ala Ala Asp
        515                 520                 525

Gly Ser Pro Gly Leu Lys Ser Val Leu Ser Thr Ser Arg Asn Leu Ser
    530                 535                 540

Asn Asn Cys Asp Thr Gly Glu Lys Pro Val Val Thr Phe Lys Glu Asn
545                 550                 555                 560

Ile Lys Thr Arg Glu Val Asn Arg Asp Gln Gly Arg Ser Phe Pro Pro
                565                 570                 575

Lys Glu Val Arg Arg Asp Tyr Ser Lys Gly Ile Thr Val Thr Lys Asn
            580                 585                 590

Asp Gly Lys Lys Asp Asn Asn Lys Arg Lys Thr Glu Thr Lys Lys Cys
        595                 600                 605

Thr Leu Glu Lys Leu Gln Glu Thr Gly Lys Gln Asn Val Ala Val Gln
    610                 615                 620

Val Lys Ser Gln Thr Glu Leu Arg Lys Thr Pro Val Ser Glu Ala Arg
625                 630                 635                 640

Lys Thr Pro Val Thr Gln Thr Pro Thr Gln Ala Ser Asn Ser Gln Phe
                645                 650                 655

Ile Pro Ile His His Pro Gly Ala Phe Pro Pro Leu Pro Ser Arg Pro
            660                 665                 670

Gly Thr Phe Leu Gln Pro Thr Ala His Ser Pro Ala Gly Asn Gln Val
        675                 680                 685

Gln Ala Gly Lys Gln Ser His Ile Pro Tyr Ser Gln Gln Arg Pro Ser
    690                 695                 700

Gly Pro Gly Pro Met Asn Gln Gly Pro Gln Gln Ser Gln Pro Pro Ser
705                 710                 715                 720

Gln Gln Pro Leu Thr Ser Leu Pro Ala Gln Pro Thr Ala Gln Ser Thr
                725                 730                 735

Ser Gln Leu Gln Val Gln Ala Leu Thr Gln Gln Gln Gln Ser Pro Thr
            740                 745                 750

Lys Ala Val Pro Ala Leu Gly Lys Ser Pro Pro His His Ser Gly Phe
        755                 760                 765

Gln Gln Tyr Gln Gln Ala Asp Ala Ser Lys Gln Leu Trp Asn Pro Pro
    770                 775                 780

Gln Val Gln Gly Pro Leu Gly Lys Ile Met Pro Val Lys Gln Pro Tyr
785                 790                 795                 800

Tyr Leu Gln Thr Gln Asp Pro Ile Lys Leu Phe Glu Pro Ser Leu Gln
                805                 810                 815

Pro Pro Val Met Gln Gln Gln Pro Leu Glu Lys Lys Met Lys Pro Phe
            820                 825                 830

Pro Met Glu Pro Tyr Asn His Asn Pro Ser Glu Val Lys Val Pro Glu
        835                 840                 845

Phe Tyr Trp Asp Ser Ser Tyr Ser Met Ala Asp Asn Arg Ser Val Met
    850                 855                 860

Ala Gln Gln Ala Asn Ile Asp Arg Arg Gly Lys Arg Ser Pro Gly Val
865                 870                 875                 880

Phe Arg Pro Glu Gln Asp Pro Val Pro Arg Met Pro Phe Glu Asp Pro
                885                 890                 895

Lys Ser Ser Pro Leu Leu Pro Pro Asp Leu Leu Lys Ser Leu Ala Ala
            900                 905                 910

Leu Glu Glu Glu Glu Glu Leu Ile Phe Ser Asn Pro Pro Asp Leu Tyr
        915                 920                 925

Pro Ala Leu Leu Gly Pro Leu Ala Ser Leu Pro Gly Arg Ser Leu Phe
    930                 935                 940

Glu Arg Tyr Pro Asn Asn Ser Met Phe Asn Glu Val Tyr Gly Lys Asn
945                 950                 955                 960

Leu Thr Ser Ser Ser Lys Ala Glu Leu Ser Pro Ser Met Ala Pro Gln
                965                 970                 975

Glu Thr Ser Leu Tyr Ser Leu Phe Glu Gly Thr Pro Trp Ser Pro Ser
            980                 985                 990

Leu Pro Ala Ser Ser Asp His Ser Thr Pro Ala Ser Gln Ser Pro His
        995                 1000                1005

Ser Ser Asn Pro Ser Ser Leu Pro Ser Ser Pro Pro Thr His Asn His
    1010                1015                1020

Asn Ser Val Pro Phe Ser Asn Phe Gly Pro Ile Gly Thr Pro Asp Asn
1025                1030                1035                1040

Arg Asp Arg Arg Thr Ala Asp Arg Trp Lys Thr Asp Lys Pro Ala Met
                1045                1050                1055

Gly Gly Phe Gly Ile Asp Tyr Leu Ser Ala Thr Ser Ser Ser Glu Ser
            1060                1065                1070

Ser Trp His Gln Ala Ser Thr Pro Ser Gly Thr Trp Thr Gly His Gly
        1075                1080                1085

Pro Ser Met Glu Asp Ser Ser Ala Val Leu Met Glu Ser Leu Lys Val
    1090                1095                1100

Ser Arg Phe Gln Glu Gln Glu Pro Gln Asp Tyr Tyr Ser Lys Val Tyr
1105                1110                1115                1120

Leu Val Gln Phe His Asp Ala Ser Trp Thr Phe Cys Ser Gly Ala Ala

1125                1130                1135

Val Asn Ala Ala Glu Ala Glu Thr Ala Thr Gly Thr Arg His His Glu
        1140                1145                1150

Pro Ser Thr Leu Arg Pro Lys Trp Gln Pro Gly Asn Glu Gly Ser Ile
    1155                1160                1165

Asn His Gly Met Leu Gly Leu Gln Asp Trp Pro Thr Gln Ser Pro Ala
  1170                1175                1180

Gly Gly Ser Pro Leu Phe Cys Phe Ser Leu Ser Arg Gly Cys Lys Tyr
1185                1190                1195                1200

Ser Thr Ser Pro Leu Ser Val His Glu Met Phe Ala Val Gln Gln Lys
                1205                1210                1215

Glu Lys Ser Ile Arg Asn Ser Pro Ser Pro Arg Gly Pro Pro Glu Gly
        1220                1225                1230

Glu Arg Glu Glu Leu Leu Phe Ile Ser Leu Ser Tyr Leu Val Ser Pro
    1235                1240                1245

Pro Leu Thr Phe Ser Ile Val His Val Pro Ser His Met Gly Ser Glu
  1250                1255                1260

Ser
1265

<210> 6
<211> 172
<212> PRT
<213> Homo sapiens

<400> 6
Met Thr Cys His His Cys Leu Leu Tyr Leu Arg Asp Leu Phe Cys Tyr
  1               5                  10                  15

Gln Arg Glu Ile Leu Pro Leu Val Thr Lys Asn Leu Ala Glu Arg Cys
             20                  25                  30

Tyr Tyr Arg Ala Leu Ser Val Ala Leu His Met Gly Met Pro Phe Asn
         35                  40                  45

Leu Leu Gly Ala Ile Thr Gly Asn Lys Tyr His Asp Ile Glu Ser Met
     50                  55                  60

Tyr Phe Tyr Gln His Trp Leu Tyr Ser Glu Val Pro Phe Lys Gly Ala
 65                  70                  75                  80

Ser Trp Ser Leu Lys Gly Leu Phe Asp His Ala Gly Lys Ser Ala Ser
                 85                  90                  95

Gly Ile Lys Tyr Lys Asp Asp Met Ser Ser Phe Phe Glu Leu Glu Pro
            100                 105                 110

Pro Thr Gly Ser Tyr Leu Gly Gln Asn Ala Ser Ile Gly Gln Phe His
        115                 120                 125

Asn Thr Leu Gln Thr Ile Lys Lys Lys Leu Lys Ile Leu Ser Val Gly
    130                 135                 140

Gly Thr Ala Ala His His Gln Gly Asp Ala Trp Leu Ala Leu Cys Gln
145                 150                 155                 160

Pro Gln Pro Pro Gln Leu Thr Leu Val Glu Ser Thr
                165                 170

<210> 7
<211> 40
<212> PRT
<213> Homo sapiens

<220>
<221> CDS
<222> (1)..(4260)

<400> 7
Lys Asp Lys Ala Lys Asp Phe Met Pro Ala Ser Lys Glu Glu Pro Ile
  1               5                  10                  15

Arg Leu Leu Arg Glu Val Val Leu Leu Thr Asp Asp Arg Asn Leu Arg
             20                  25                  30

Val Lys Ala Leu Thr Arg Asn Val
         35                  40

<210> 8
<211> 40
<212> PRT
<213> Homo sapiens

<220>
<221> PEPTIDE
<222> (1)..(40)

<400> 8
Lys Ser Phe Glu Arg His Lys Leu Lys Arg Gln Asp Ala Asp Ala Trp
  1               5                  10                  15

Thr Leu Tyr Lys Ile Leu Asp Ser Cys Lys Gln Leu Thr Leu Ala Gln
             20                  25                  30

Gly Ala Gly Glu Glu Asp Pro Ser
         35                  40

## Description

The present invention relates to a novel proteins, peptide fragments and conjugates thereof, and nucleic acid encoding for the proteins and fragments, that have utility in modulation of telomere production. The novel protein, peptide fragments and DNA have utility in rendering cells capable of longer or shorter replicative life, at least in terms of number of replications before the cell exits the cell cycle, and in screening for agents that are capable of anticancer activity in so far as they modulate a cells ability to 'immortalise' itself.

Telomerase is a ribonucleoprotein particle required for the complete replication of chromosome ends (see Curr. Op. Genetics and Development 8, 226 (1998). The telomerase RNA moiety is tightly associated with telomerase proteins and serves as a template for telomerase repeat synthesis. Telomerase activity is absent in normal human somatic cells and therefore, chromosomal ends or telomeres shrink with each replication cycle in these cells (see Science 279, 349 (1998)). This deficit limits the replicative potential of normal human somatic cells. In 85% of all tumours telomerase is upregulated and telomerase activation appears to be a critical event during tumorigenesis (see Nature 400 464 (1999)). Telomerase inhibitors are of potential use for the treatment of neoplasias while the absence of telomerase activity in most normal tissues has been implicated in age-related diseases.

Yeast Est1p is a yeast telomerase component essential for the replication of telomeres. Upon deletion of the yeast *EST1* gene, telomeres shrink with each replication cycle, as would be the case in a telomerase defect (see Cell 57, 633 (1989)). Est1p is associated with yeast telomerase RNA and binds to single-stranded telomerase DNA *in vitro* (see Current Biology 10, 809 (2000)). It is thought to mediate the interaction between the telomere and telomerase (Proc. Natl. Acad. Sci 94, 11190 (1997); Science 286, 117 (1999).

The present inventors have identified human cDNA sequences that can be demonstrated to have low but significant sequence homology to yeast EST1 (see Table 1 and Figures 1 and 2 herein). Using reverse-transcriptase PCR (RT-PCR) they have amplified the cDNA sequences missing in deposited sequences and derived the complete open reading frames. Northern blot analysis (see Figure 3 herein) demonstrates that these homologues are expressed in normal cells and in tumour derived cell lines (see Figure 3).

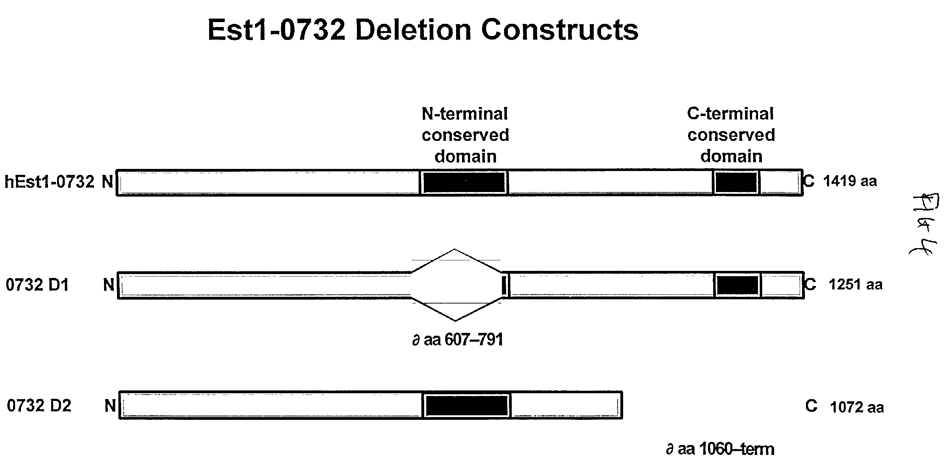

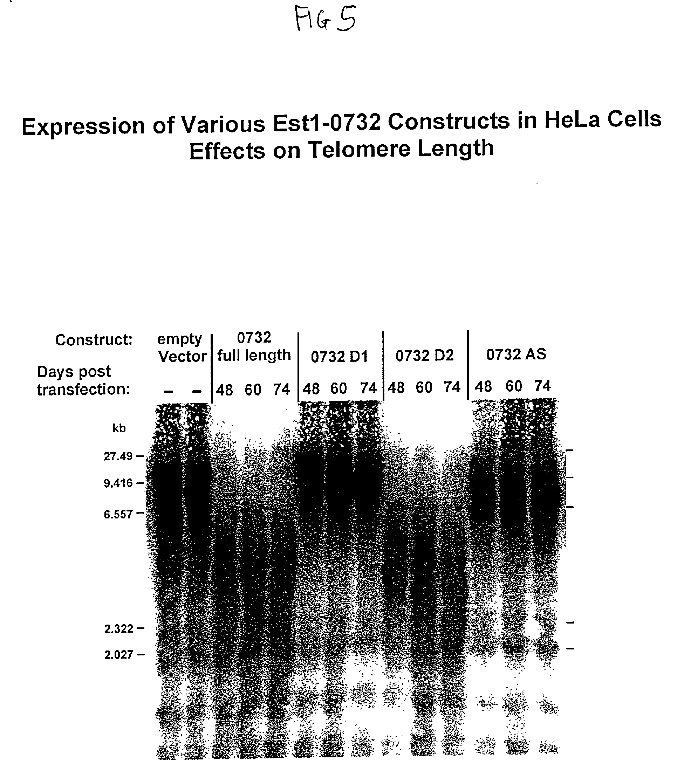

The inventors have further provided antibodies against synthetic peptides predicted from the gene and protein sequences (see Figure 4) and with one of these were able to immunoprecipitate the telomerase RNA moiety, demonstrating that this protein is associated with telomerase *in vivo* (see Table 2). Furthermore, they have demonstrated that the antibody specific for this protein also immunoprecipitates telomerase activity (see Figure 5) which demonstrates that the protein is associated with active telomerase *in vivo.*

The inventors have still further demonstrated that, by expressing this protein in a human derived tumour cell line having telomerase activity, it is possible to interfere with native telomerase activity such that telomeres are reduced in length and thus their replicative capacity is reduced. This is probably, but not exclusively, effected by recruitment of other telomerase obligate components away from the telomerase reverse transcriptase active subunit (hTERT or EST-2) by the overexpressed EST1.

Thus in a first aspect of the present invention there is provided an isolated, enriched, purified or recombinant nucleic acid sequence encoding for a protein having activity as a human telomerase activity modulator characterised in that it comprises a nucleotide sequence encoding for an amino acid sequence selected from those of SEQ ID No 2, SEQ ID No 4, SEQ ID No 5 and SEQ ID No 6 and sequences having at least 70% amino acid homology thereto and an active peptide fragment of any of these sequences.

More preferably the protein comprises an amino acid sequence of SEQ ID No 2 or 4 or one that has 75% or more homology, more preferably 80% or more and still more preferably 90% or more to one of those sequences.

More preferably the nucleic acid comprises a nucleic acid sequence selected from SEQ ID No 1 or SEQ ID No 3 or is capable of hybridising with nucleic acid having such a sequence under conditions of defined stringency.

Preferred nucleotide sequences include those that will hybridize with those of SEQ ID No 1 or 3 under standard stringency conditions of 2 x SSC, more preferably high stringency conditions of 1 x SSC (see Church and Gilbert, Proc Nat Acad Sci USA (1984) 81, 1991-1995 incorporated herein by reference).

Preferred fragments, particularly of SEQ ID No 1 and homologues are C-terminally truncated fragments.

It will be realised that by active fragments and homologues having the stated homology thereto we intend functional variants. A "functional variant" of a peptide or protein is a polypeptide the amino acid sequence of which can be derived from the amino acid sequence of the original peptide or protein by the substitution, deletion and/or addition of one or more amino acid residue in a way that, in spite of the change in the amino acid sequence, the functional variant retains at least a part of at least one of the biological activities of the original protein that is detectable for a person skilled in the art.

Preferably the amino acid sequence of the functional variant is 50% identical, more preferably 70% identical and most preferably 90% identical to the peptide or protein. Any functional part of a protein or a variant thereof is also termed functional variant. Biological function in the present application is the ability to modulate human telomerase activity *in vivo* or the activity of hTERT *in vitro* or *in vivo.*

By homologous is meant that the stated percentage of the amino acid sequence has identity or is of conservatively substituted amino acids. By identical is meant that the stated percentage of the amino acid sequence is identical. Both these percentage terms allow for gapping of sequences to allow alignment as is described below.

Particularly, by the term identity is meant that the stated percentage of the claimed amino acid sequence or base sequence is to be found in the reference sequence in the same relative positions when the sequences are optimally aligned, notwithstanding the fact that the sequences may have deletions or additions in certain positions requiring introduction of gaps to allow alignment of the highest percentage of amino acids or bases. Preferably the sequence are aligned by using 20 or less gaps, ie. the total number of gaps introduced into the two sequences when added together is 20 or less, more preferably 10 or less. The length of such gaps is not of particular importance as long as the defined activity relating to modulation is maintained but generally will be no more than 50, and preferably no more than 10 amino acids, or 150 and preferably no more than 30 bases.

Variants from the aforesaid sequences preferably are conservative substitutions. The expression 'conservative substitutions' as used with respect to amino acids relates to the substitution of a given amino acid by an amino acid having physicochemical characteristics in the same class. Thus where an amino acid has a hydrophobic characterising group, a conservative substitution replaces it by another amino acid also having a hydrophobic characterising group; other such classes are those where the characterising group is hydrophilic, cationic, anionic or contains a thiol or thioether. Such substitutions are only contemplated where the resultant protein has hTERT modulating activity. For the present purposes proline may be treated as hydrophobic, but is preferably not substituted.

Algorithms and software suitable for use in aligning amino acid or nucleotide sequences for comparison and calculation of sequence homology or identity will be known to those skilled in the art. Significant examples of such tools are the Pearson and Lipman search based FAST and BLAST programs. Details of these may be found in Altschul et al (1990), J. Mol. Biol. 215: 403-10; Lipman D J and Pearson W R (1985) Science 227, p1435-41. Publicly available details of BLAST may be found on the internet at 'http://www.ncbi. nlm.nih.gov/BLAST/blast-help.html'. Thus such homology and identity percentages can be ascertained using commercially or publicly available software packages incorporating, for example, FASTA and BLASTn software or by computer servers on the internet. Examples of the former are the GCG program package (Devereux et al Nucleic Acids Research (1984) 12 (1): 387) and the Bestfit program (Wisconsin Sequence Analysis Package, eg. Version 8 for Unix or IBM equivalent, Genetics Computer Group, University Research Park, 575 Science Drive, Madison, WI 53711 ) which uses the local homology algorithm of Smith and Waterman, Advances in Mathematics 2:482-489 (1981). Many international institutes, eg. Genbank (see http://www.ncbi.nlm.nih.gov/BLAST) and EMBL: (see http://www.emb1-heidelberg.de/Blast2), offer internet services.

Parameters used in with software packages and internet servers should be applied with the appropriate sequence lengths and aforesaid gap characteristics in mind. Alignment strategies are discussed further in WO 98/40483 on pages 39 to 41, which document is incorporated herein by reference

Convenient parameters for BLAST searches are the default values, ie. for EMBL Advanced Blast2: Blastp Matrix BLOSUMS 62, Filter default, Echofilter X, Expect 10, Cutoff default, Strand both, Descriptions 50, Alignments 50. For BLASTn defaults are again preferably used. GCG Wisconsin Package defaults are Gap Weight 12, Length weight 4. FASTDB parameters used for a further preferred method of homology calculation are mismatch penalty = 1.00, gap penalty =1.00, gap size penalty = 0.33 and joining penalty =30.0.

Nucleic acids of the invention may be degeneratively substituted with respect to that exemplified herein in the sequence listing. The expression 'degeneratively substituted' refers to substitutions of nucleotides by those which result in codons encoding for the same amino acid; such degenerative substitutions being advantageous where the cell or vector expressing the protein is of such different type to the DNA source organism cell that it has different codon preferences for transcription/translation to that of the cDNA source cell. Such degenerative substitutions will thus be host specific

Preferred encoded proteins may be provided as conjugated to other proteins, eg chromosome end capping proteins or peptides eg. such as cdc13, as fusion proteins and the nucleic acid, eg DNA, may encode for such fusions.

In a second aspect of the invention there is provided an isolated, enriched, purified or recombinantly produced protein comprising an amino acid sequence selected from the group consisting of SEQ ID No 2, SEQ ID No 4, SEQ ID no 5 and SEQ ID No 6 or a part of any of these, the protein or part thereof being effective to modulate the activity of telomerase in tumour cells *in vivo* or the ability of hTERT (human EST2) to extend telomere length *in vitro.*

It will be realised that one way to determine the ability of the protein to modulate the activity of telomerase *in vivo* will involve expressing nucleic acid, eg. DNA, within the tumour cell and comparing the length of telomeres in that cell with that of telomeres in a cell of similar origin that lacks that expression of nucleic acid.

In a third aspect of the present invention there is provided antisense nucleic acid, particularly DNA, to that of the first aspect such that it is capable of downregulating expression of one or more of human EST1 gene products *in vivo* such that telomerase activity in a cell, particularly a tumour or precancerous cell, is reduced or eliminated. In this manner an 'immortalised' cancer or precancerous cell, eg. a cell that would but for other anti-neoplastic agents, eg. p53 or pRb, be cancerous, can be limited in replicative potential.

In a fourth aspect of the present invention there is provided an method for screening for agents suspected of having mammalian, particularly human, telomerase modulating activity, *in vivo* or *in vitro,* characterised in that it comprises (i) placing hTERT and a protein of the second aspect together in an aqueous medium together with DNA, telomerase RNA and the suspect agent, (ii) monitoring the ability of the hTERT (human EST2) to produce or extend telomeres on the DNA, (iii) determining any modulation of the elongation of telomeres in response to the agent as compared to a control medium having hTERT, protein, DNA, telomerase RNA but lacking the agent and (iv) designating the agent as a suspected inhibitor or activator of telomerase activity or as inactive accordingly.

It will be realised that the assay medium may further include a human end capping protein, such as cdc13 protein, and any other cofactors that might be of utility in such a screening method. The method may also be carried out in a cell line lacking human telomerase activity.

In a fifth aspect of the present invention there is provided a method of screening for agents suspected of having mammalian, particularly human, *in vivo or in vitro* telomerase modulating activity characterised in that it comprises (i) providing one or more of human telomerase (hTERT or human EST2) and telomerase RNA together in an assay medium or cell together with a protein or fragment thereof of the second aspect and the suspected agent, (ii) lysing the cell if present and treating the lysate to provide a fraction containing the protein or fragment, (iii) mixing the fraction or medium containing the protein or fragment together binding agent specific for binding the protein or fragment and (iv) assessing the content of bound fragment from step (iii) for telomerase and/or telomerase RNA content and (v) correlating the amount of bound telomerase activity or RNA with activity of the suspect agent as either an inhibitor or activator of interaction of human *in vivo* telomerase activity or an inactive accordingly.

This aspect is conveniently carried out by use of a pull down assay with an antibody raised to the protein or fragment of the second aspect of the invention.

In a sixth aspect of the present invention there is provided an antibody capable of binding a protein or peptide of the second aspect in a preferred or specific manner. Such antibody may be monoclonal or polyclonal, immobilised or free and is exemplified in the examples below as used in a pull down assay.

In a seventh aspect of the present invention there is provided a method of providing or enhancing human telomerase activity in a cell that lacks or has relatively low native human telomerase activity comprising recombinantly incorporating nucleic acid, particularly DNA, as claimed in the first aspect of the invention into the cell in functional relationship with a promoter active in said cell.

Preferably the cell is a human cell for which it is desired to increase, potentially indefinitely, the number of potential cell replication cycles. In this manner so called 'immortalisation' of cells of benefit can be provided. For example, human somatic cells of a type particularly suited to the production of a particular therapeutic or diagnostic peptide or protein may be enhanced with respect to replicative potential, thus rendering them more useful in large laboratory scale or industrial scale production of said peptide or protein.

It will be realised that recombinantly incorporating only nucleic acid, eg. DNA, of the first aspect may not always be sufficient, and thus preferred methods will also include incorporation of one or more nucleic acids encoding human EST2 (hTERT), human EST3 and cdc13 or some other protein having teleomere end capping function in humans. It may also involve expressing conjugations of the protein of the invention together with one or more of these, particularly a conjugate of the present hEST1 with human cdc13 or a protein having telomere end capping function in humans.

Promoters active in a human cell will occur to those skilled in the art and include CMV, SV40 and mPSV promoters.

In an eight aspect of the present invention there is provided a vector or recombinant DNA encoding a dominant negative mutant EST1 protein or fragment of the second aspect of the invention sufficient to inhibit telomerase subunit assembly or binding to teleomeres when expressed in a cell, particularly a tumour cell.

In a ninth aspect of the present invention there is provided a method of treating a mammalian patient in need of therapy for a tumour which has telomerase activity comprising transforming the tumour cells with a vector comprising DNA of the eight aspect. Preferred vectors may be in the form of a viral vector, such as those of adenovirus, lentivirus, adenovirus associated virus (AAV), parvovirus, herpes virus and other suitable gene therapy vectors. Particularly preferred are those viral vectors that include tumour or tissue specific promoters such as those referred to in PCT/US95/15455 or PCT/GB00/01142 incorporated herein by reference. Alternatively the transformation may be provided by integrating the nucleic acid, eg DNA, of the invention behind an existing promoter within a target cell.

In a tenth aspect of the present invention there is provided a method of treating a mammalian patient in need of therapy for a disease wherein cells are losing the ability to replicate with resultant deficit in tissue function characterised in that it comprises transforming one or more of these cells with nucleic acid of the seventh aspect such that it is in functional relationship with a promoter functional in said cells. Again this may be carried out using a vector such as a viral vector or may be carried out by directly integrating the DNA into the cell, and most advantageously may be integrated functionally with respect to an existing endogenous promoter.

In an eleventh aspect the present invention provides a vector, such as a viral vector described above in the ninth aspect, comprising a nucleic acid and optional promoter as described in the tenth aspect.

The method of the tenth aspect and vector of the eleventh aspect have particular application to diseases of ageing and wasting diseases.

A twevlth aspect of the present invention provides PCR primers comprising from 10 to 30 contiguous bases of the sequence SEQ ID No 1 or SEQ ID No 3 or complementary sequence thereto.

A thirteenth aspect of the present invention provides an oligonucleotide probe, having use in detecting nucleic acid of the invention, comprising from 15 to the full length contiguous sequence of SEQ ID No 1 or SEQ ID No 3. Preferably the probe is labelled, eg. with radiolabelled phosphorous.

The present invention will now be described further by way of illustration only by reference to the following non-limiting examples, Figures and Sequence listing. Further embodiments of the invention falling within the scope of the claims will occur to those skilled in the art in the light of these.

### SEQUENCE LISTING

SEQ ID No 1 is the nucleic acid sequence of a cDNA encoding a preferred protein of the invention, designated herein EST1-0732.
SEQ ID No 2 is the amino acid sequence of that preferred protein of the invention.
SEQ ID No 3 is the nucleic acid sequence of a cDNA encoding a second protein of the invention, designated herein EST1-1089.
SEQ ID No 4 is the amino acid sequence of that protein of the invention.
SEQ ID No 5 is the amino acid sequence of a further protein of the invention designated herein EST1-3.
SEQ ID No 6 is the amino acid sequence of a further protein of the invention designated herein EST1-4.
SEQ ID No 7 is the amino acid sequence of a peptide fragment used as an antigen to raise antibodies specific to the protein of SEQ ID No 2.
SEQ ID No 8 is the amino acid sequence of a peptide fragment used as an antigen to raise antibodies specific to the protein of SEQ ID No 4.

### FIGURES

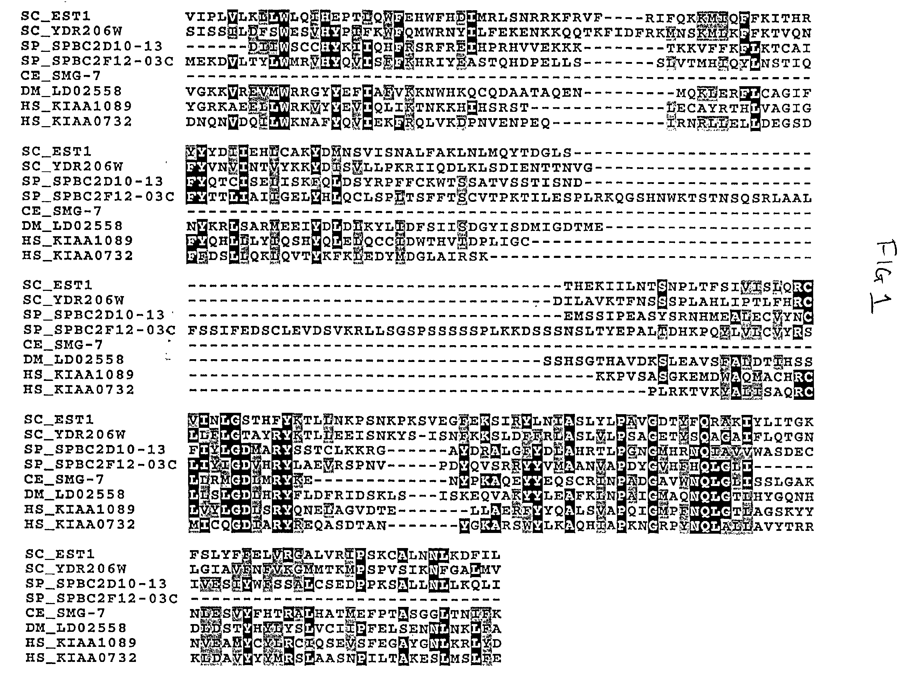

Figure 1 is an alignment of the amino acid sequences of SEQ ID No 2 (0732) and 4 (1089) with those of other EST1 proteins S. cerevisiae EST1 and YDR206W, S. plombe SPBC2D10-13 and SPBC2F12-03C, C. elegans SMG-7, D. melanogastar LD02558.

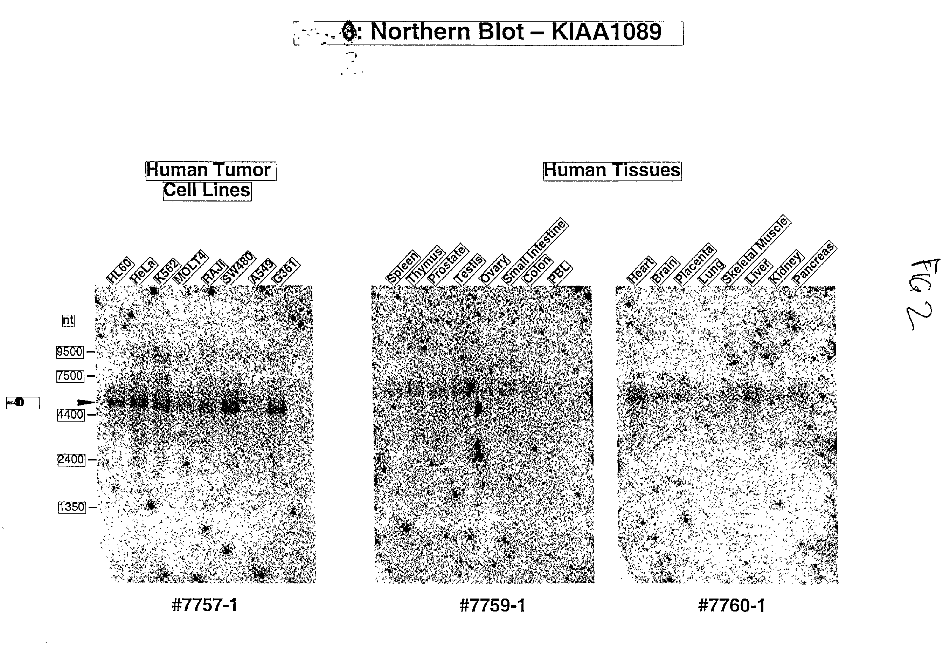

Figure 2 is a Northern Blot for the DNA of SEQ ID No 3.

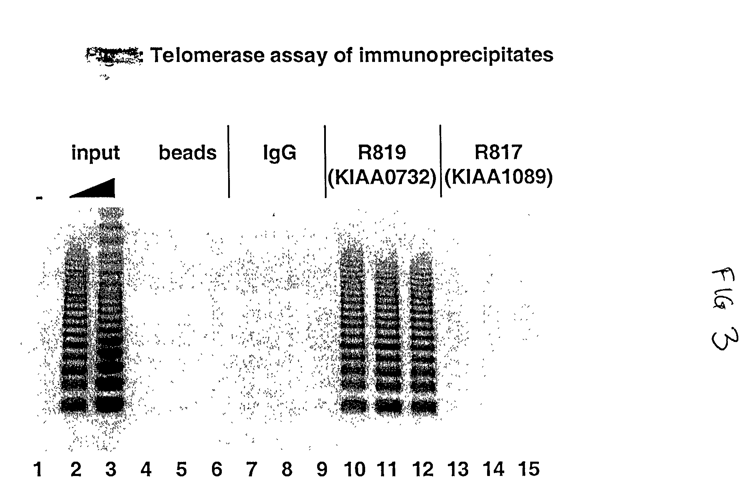

Figure 3 shows a telomerase assay of immunoprecipitates produced using antibodies to the proteins of SEQ ID No 2 (0732) and 4 (1089).

Figure 4 shows diagrammatic representation of the constructs used in HeLa cell studies of Example 5.

Figure 5 shows the effect of these studies on HeLa cell telomere length.

### EXAMPLES

### Example 1.

### Production of cDNAs encoding SEQ ID No 2 and 4.

DNA was produced using 5'RACE technique using human genomic RNA as template and a suitable primers targeted at the ends of the sequences shown in the sequence listing. Similar techniques were used to obtain the sequences encoding SEQ ID No 5 and 6.

### Example 2.

### Expression of human EST1 proteins of SEQ ID No 2 and 4.

Northern blots were produced from RNA fractions derived from a variety of different human tissues and probed using radiolabelled oligonucleotide probes specific to DNA of SEQ ID No 1 and SEQ ID No 3. Figure 2 shows the distribution of the mRNA encoding SEQ ID No 3 while similar distribution was found for SEQ ID No 1 (not shown).

### Example 3.

### Production of antibodies to proteins comprising SEQ ID No 2 and SEQ ID No 4.

Peptides of 40 amino acids length were designed for each of the four homologues SEQ ID No 2, 4, 5 and 6. 500 µg of each peptide were used to immunise two rabbits per peptide, following the Eurogentec Bel S.A. (Herstal, Belgium) standard protocol. Intradermic multisite injections of 100 µg peptide (500 µl antigen solution, 500 µl adjuvant) were performed on days 0, 14, 28 and 56. Test blood samples (days 38 and 66) were checked for affinity to the peptides. All antibodies were affinity purified on antigen columns as described in Sambrook, J., Fitsch, E. F. and Maniatis, T. (1989). Molecular cloning, a laboratory manual, Cold Spring Harbor, New York (incorporated herein by reference). The peptide an antibody was raised against was coupled to NHS-activated Sepharose 4 Fast Flow (Pharmacia) and washed extensively at low and high pH. Blood samples (20 ml) of immunized rabbits were passed over these antigen columns, the columns were washed, and bound antibodies eluted at high and low pH. Elution fractions were combined, concentrated and tested for antigen affinity in western blots.

### Example 4.

### Telomerase assay (TRAP) of immunoprecipitates.

As shown in Figure 3, HeLa cell nuclear extract (Lanes 1,2) was incubated with Protein A sepharose beads in the absence of antibody (lanes 4-6), presence of antibody to protein of SEQ ID No 7 (lanes 10-12) and antibody to protein of SEQ ID No 8 (lanes 13- 15) and IgG negative control (lanes 7-9). The experiment was done in triplicate. Telomerase activity was specifically immunoprecipitated with antibody to SEQ ID No 7 (and thus SEQ ID No 2) demonstrating the association of 0732 (SEQ ID No 2) with active telomerase.

### Example 5.

### Expression of Est1-0732 constructs in HeLa cells - effects on telomere length.

Various Est1-0732 (SEQ ID No 2) constructs were overexpressed in HeLa cells I order to determine whether they had any in vivo activity with respect to telomere length.

Constructs used were as follows:
pCMV0732: Full length open reading frame of Est1-0732 in pEGFP-C2 (Clontech) from which GFP was removed by cutting the pEGFP-C2 vector with NheI and SmaI. The promoter used to drive over-expression was the CMV promoter.
0732 D1: pCMV0732 was cut with Acc III and Fsp I, filled in to blunt end with Klenow enzyme in the presence of dNTPs and religated. This leads to a deletion of amino acids 607-791, which correspond to the region that is conserved among the Est1-family members shown in the alignment of Figure 1.
0732 D2: pCMV0732 was cut with Sal I and religated. This leads to deletion of amino acids 1060-end.

Transfection of HeLa cells:
HeLa cell were transfected using the GenePORTER transfection reagent (from AXON LAB AG). Cells were selected on neomycin and grown under standard conditions.

Figure 4 shows diagrammatic representation of these constructs while Figure 5 shows the effect of transfecting the cells on telomere length, with the empty vector, D1 and AS having little or no effect but full length SEQ ID No 1 and D2 vectors showing clear reduction in telomere length, providing evidence that SEQ ID no 1 and C-terminal truncates thereof have activity in recruiting components of the telomerase obligate components away from hTERT.

## Claims

1. An isolated, enriched, purified or recombinant nucleic acid sequence encoding for a protein having activity as a human telomerase activity modulator **characterised in that** it comprises a nucleotide sequence encoding for an amino acid sequence selected from those of SEQ ID No 2, SEQ ID No 4, SEQ ID No 5 and SEQ ID No 6 and sequences having at least 70% amino acid homology thereto and an active peptide fragment of any of these sequences.

2. A nucleic acid as claimed in Claim 1 encoding a protein comprising an amino acid sequence of SEQ ID No 2 or 4 or one that has 75% or more homology, more preferably 80% or more and still more preferably 90% or more to one of those sequences.

3. A nucleic acid as claimed in Claim 1 or 2 comprising a nucleic acid sequence selected from SEQ ID No 1 or SEQ ID No 3 or which is capable of hybridising with nucleic acid having such a sequence under conditions of standard or high stringency.

4. A nucleic acid as claimed in any one of the preceding claims encoding for a fragment of said protein **characterised in that** it is a C-terminally truncated fragment of said protein.

5. A nucleic acid as claimed in any one of the preceding claims **characterised in that** it encodes for a protein as described in any one of the preceding claims conjugated to other proteins, eg chromosome end capping proteins or peptides eg. such as cdc13, as fusion proteins.

6. An isolated, enriched, purified or recombinantly produced protein comprising an amino acid sequence selected from the group consisting of SEQ ID No 2, SEQ ID No 4, SEQ ID no 5 and SEQ ID No 6 or a part of any of these, the protein or part thereof being effective to modulate the activity of telomerase in tumour cells *in vivo* or the ability of hTERT (human EST2) to extend telomere length *in vitro.*

7. A protein or part as claimed in Claim 6 as encoded by any one of the nucleic acids of Claims 1 to 5.

8. Antisense nucleic acid to nucleic acid as claimed in any one of Claims 1 to 5.

9. Antisense nucleic acid as claimed in Claim 8 **characterised in that** it is capable of downregulating expression of one or more of human EST1 gene products *in vivo* such that telomerase activity in a cell in which it is provided, particularly a tumour or precancerous cell, is reduced or eliminated.

10. A method for screening for agents suspected of having mammalian, particularly human, telomerase modulating activity, *in vivo* or *in vitro,* **characterised in that** it comprises (i) placing hTERT and a protein of the second aspect together in an aqueous medium together with DNA, telomerase RNA and the suspect agent, (ii) monitoring the ability of the hTERT (human EST2) to produce or extend telomeres on the DNA, (iii) determining any modulation of the elongation of telomeres in response to the agent as compared to a control medium having hTERT, protein, DNA, telomerase RNA but lacking the agent and (iv) designating the agent as a suspected inhibitor or activator of telomerase activity or as inactive accordingly.

11. A method of screening for agents suspected of having mammalian, particularly human, *in vivo or in vitro* telomerase modulating activity **characterised in that** it comprises (i) providing one or more of human telomerase (hTERT or human EST2) and telomerase RNA together in an assay medium or cell together with a protein or fragment thereof of the second aspect and the suspected agent, (ii) lysing the cell if present and treating the lysate to provide a fraction containing the protein or fragment, (iii) mixing the fraction or medium containing the protein or fragment together binding agent specific for binding the protein or fragment and (iv) assessing the content of bound fragment from step (iii) for telomerase and/or telomerase RNA content and (v) correlating the amount of bound telomerase activity or RNA with activity of the suspect agent as either an inhibitor or activator of interaction of human *in vivo* telomerase activity or an inactive accordingly.

12. An antibody capable of binding a protein or peptide as claimed in Claim 6 or Claim 7.

13. A method of providing or enhancing human telomerase activity in a cell that lacks or has relatively low native human telomerase activity comprising recombinantly incorporating nucleic acid, particularly DNA, as claimed any one of Claims 1 to 5 into the cell in functional relationship with a promoter active in said cell.

14. A vector or recombinant DNA encoding a dominant negative mutant EST1 protein or fragment as claimed in Claim 6 or 7 sufficient to inhibit telomerase subunit assembly or binding to teleomeres when expressed in a cell, particularly a tumour cell.

15. A method of treating a mammalian patient in need of therapy for a tumour which has telomerase activity comprising transforming the tumour cells with a vector comprising DNA as claimed in Claim 14.

16. A method of treating a mammalian patient in need of therapy for a disease wherein cells are losing the ability to replicate with resultant deficit in tissue function **characterised in that** it comprises transforming one or more of these cells with nucleic acid as claimed in any one of Claims 1 to 5 such that it is in functional relationship with a promoter functional in said cells.

17. A vector, such as a viral vector comprising a nucleic acid and optional promoter as claimed in any one of Claims 1 to 5.

18. A PCR primers comprising from 10 to 30 contiguous bases of the sequence SEQ ID No 1 or SEQ ID No 3 or complementary sequence thereto.

19. An oligonucleotide probe comprising from 15 to the full length contiguous sequence of SEQ ID No 1 or SEQ ID No 3 or complementary sequence thereto.
